(19)

Europäisches
Patentamt
European
Patent Office
Office européen
des brevets

(11) **EP 4 159 870 B1**

(12) **EUROPEAN PATENT SPECIFICATION**

(45) Date of publication and mention
of the grant of the patent:
**07.05.2025 Bulletin 2025/19**

(51) International Patent Classification (IPC):
**C12Q 1/02** (2006.01)          **C12Q 1/04** (2006.01)

(52) Cooperative Patent Classification (CPC):
**C12Q 1/02; C12Q 1/045**

(21) Application number: **22199579.8**

(22) Date of filing: **04.10.2022**

(54) **SYSTEM FOR THE DETECTION OF INHIBITORS IN MILK**

SYSTEM ZUM NACHWEIS VON INHIBITOREN IN MILCH

SYSTÈME DE DÉTECTION D'INHIBITEURS DANS LE LAIT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.10.2021 EP 21200810**

(43) Date of publication of application:
**05.04.2023 Bulletin 2023/14**

(73) Proprietor: **Analytik in Milch Produktions- und
Vertriebs-GmbH
80336 Munich (DE)**

(72) Inventor: **SCHAUER, Kristina
85764 Oberschleissheim (DE)**

(74) Representative: **Grünecker Patent- und
Rechtsanwälte
PartG mbB
Leopoldstraße 4
80802 München (DE)**

(56) References cited:
**FR-A1- 2 882 370**

- **MUHAMMAD SYED AUN ET AL: "Production and
characterization of a new antibacterial peptide
obtained from Aeribacillus pallidus SAT4",
BIOTECHNOLOGY REPORTS, vol. 8, 21
September 2015 (2015-09-21), pages 72 - 80,
XP093025731, ISSN: 2215-017X, Retrieved from
the Internet <URL:https://www.ncbi.nlm.nih.gov/
pmc/articles/PMC4980744/pdf/main.pdf> DOI:
10.1016/j.btre.2015.09.003**

- **HARIRCHI SHARAREH ET AL: "Efficacy of
polyextremophilic Aeribacillus pallidus on
bioprocessing of beet vinasse derived from
ethanol industries", BIORESOURCE
TECHNOLOGY, ELSEVIER, AMSTERDAM, NL,
vol. 313, 12 June 2020 (2020-06-12),
XP086219336, ISSN: 0960-8524, [retrieved on
20200612], DOI: 10.1016/
J.BIORTECH.2020.123662**

- **GUNA REMEDIOS ET AL: "In vitro activity of
linezolid, clarithromycin and moxifloxacin
against clinical isolates of Mycobacterium
kansasii", JOURNAL OF ANTIMICROBIAL
CHEMOTHERAPY, vol. 55, no. 6, 1 June 2005
(2005-06-01), GB, pages 950 - 953, XP093025778,
ISSN: 0305-7453, Retrieved from the Internet
<URL:https://watermark.silverchair.com/dki111.
pdf?
token=AQECAHi208BE49Ooan9kkhW_Er
cy7Dm3ZL_9Cf3qfKAc485ysgAAAtQwggLQBgk
qhkiG9w0BBwagggLBMIICvQIBADCCArYGCSq
GSIb3DQEHATAeBglghkgBZQMEAS4wEQQM7r
aTUMphugq7TIKqAgEQgIICh1Vps1IJyjdqXpzo-1
jiBAHvYGJAXr2q2-PAo_qODpotG-byyxP5n
NuUypNbusft_vyCO5IgFvIUGP5_mb
VEyjcM4_W0F> DOI: 10.1093/jac/dki111**

- **C. SALA ET AL: "Simple Model for Testing Drugs
against Nonreplicating Mycobacterium
tuberculosis", ANTIMICROBIAL AGENTS AND
CHEMOTHERAPY, vol. 54, no. 10, 1 October 2010
(2010-10-01), US, pages 4150 - 4158,
XP055456289, ISSN: 0066-4804, DOI: 10.1128/
AAC.00821-10**

**(Cont. next page)**

EP 4 159 870 B1

- WU QIN ET AL: "A Novel Microbiological Method in Microtiter Plates for Screening Seven Kinds of Widely Used Antibiotics Residues in Milk, Chicken Egg and Honey", FRONTIERS IN MICROBIOLOGY, vol. 10, 12 March 2019 (2019-03-12), XP093025297, DOI: 10.3389/fmicb.2019.00436
- ANONYMOUS: "embedded", OXFORD ENGLISH DICTIONARY, 1 January 2024 (2024-01-01), XP093240684, Retrieved from the Internet <URL:https://www.oed.com/search/dictionary/?scope=Entries&q=embedded>
- ANONYMOUS: "kit API 20 E", 1 January 2002 (2002-01-01), XP093240686, Retrieved from the Internet <URL:https://biomanufacturing.org/uploads/files/587872707301898351-api20einstructions.pdf>
- ANONYMOUS: "kit API 50CHB/E Medium", 1 January 2011 (2011-01-01), XP093240685, Retrieved from the Internet <URL:https://www.scribd.com/doc/35548478/api50-CHB-E-medium>

## Description

### CROSS-REFERENCE TO RELATED APPLICATIONS

This application claims priority to earlier European patent application EP 21 200 810.6, filed on October 4, 2021.

### TECHNICAL FIELD

The present disclosure generally relates to microbial test systems for detecting inhibitors in milk. More specifically this disclosure relates to a microbial test system for detecting an inhibitor in milk comprising indicator bacteria of the species *Aeribacillus pallidus* embedded in a growth medium, the use of said indicator bacteria as well as a method, a test kit and a growth medium for detecting an inhibitor in milk.

### BACKGROUND OF THE INVENTION

Antibiotics are a group of inhibitors, which inhibit bacterial growth, and are used for the treatment of bacterial infections in dairy animals, such as cattle, buffaloes, goats, sheep and camels. The use of these inhibitors (e.g. antibiotics) may lead to contamination of raw milk products with the inhibitors by secretion of the inhibitor into the milk or for post-secretory reasons. In particular, in the case of systemically applied antibiotics, the raw milk may be contaminated with the antibiotics, which may be secreted in the milk of lactating animals, such as dairy animals.

The applied antibiotics may be a quinolone antibiotic. Most quinolone antibiotics that are currently used are fluoroquinolones.

Currently three fluoroquinolones, enrofloxacin (ENR), marbofloxacin (MAR) and danofloxacin (DAN), all available in the form of various mono-preparations, are used for therapeutic application in dairy cattle in Germany. Fluoroquinolones have good pharmacokinetic properties for systemic administration and a wide therapeutic range.

Fluoroquinolones in dairy cattle are used to treat bacterial infections in various organ systems caused by fluoroquinolone-sensitive strains of mainly gram-negative and atypical pathogens, for example in the treatment of respiratory diseases caused by *Pasteurella multocida, Mannheimia haemolytica, Histophilus somni* and *Mycoplasma bovis,* in severe mastitis, enteritis or septicaemia caused by *Escherichia coli,* as well as in the treatment of arthritis caused by *Mycoplasma bovis.*

After administration of fluoroquinolones, waiting periods of at least 36 hours to five days after the last administration of the fluoroquinolone have to be observed until the use of milk of the respective animal. The specific duration of the waiting period depends on the active ingredient, the dosage and the route of administration. In lactating animals, fluoroquinolones are also secreted into the milk.

One problem using fluoroquinolones in lactating animals, such as dairy cattle, is that fluoroquinolones are highly resistant to thermal treatment. For example, milk samples containing ciprofloxacin or enrofloxacin show a degradation of 12% and 5%, respectively, after heating for 20 min at 120 °C. Accordingly, the persistence of fluoroquinolones in processed dairy products is considered to be high. For example, fluoroquinolones are detected at high concentrations in yogurt and hard cheese made from goat milk spiked with fluoroquinolones, even after 30 days and six months of storage. Fluoroquinolone contaminated milk may have potential effects and risks on consumer health, technological processes in milk processing, and the development of resistance to environmentally and food-associated bacteria. Consequently, the prevention and detection of fluoroquinolone residues in milk is of major importance.

Raw milk quality and residual amounts of inhibitors are strictly regulated. Conditions for the use of inhibitors in food-producing animals, such as dairy animals and requirements for monitoring residual amounts of inhibitors in corresponding products, such as raw milk, are set by law.

Commission Regulation (EC) 853/2004 of 29 April 2004 lays down specific hygiene rules for food of animal origin, e.g. it prohibits the marketing of raw milk containing antibiotic residues if maximum residue levels (MRL) are exceeded. Maximum residue limits of inhibitors in animal derived foodstuffs, for instance milk, are set by the Commission Regulation (EU) 37/2010 of 22 December 2009 on pharmacologically active substances and their classification regarding maximum residue limits in foodstuffs of animal origin.

For instance, in Table 1 of the Annex of Commission Regulation (EU) 37/2010, the maximum residue levels (MRLs) of various fluoroquinolones have been set to be 100 µg/kg for the sum of enrofloxacin (ENR) and its metabolite ciprofloxacin (CIP) in milk of bovine, ovine and caprine species, 30 µg/kg for danofloxacin (DAN) in milk of bovine, ovine and caprine species, 75 µg/kg for marbofloxacin (MAR) in milk of bovine species and 50 µg/kg for flumequine (FLU) in milk of bovine, ovine and caprine species. Oxolinic acid, flumequine and difloxacin are not permitted for use in animals from which milk is produced for human consumption, in particular they do not have a marketing authorization in Germany.

According to Regulation (EU) 2017/625 of 15 March 2017 and Commission Decision 97/747/EC of 27 October 1997, a certain number of raw milk samples has to be tested annually in each European Union member state for the presence of inhibitors, such as antibiotically active veterinary medicinal products of different substance classes, as part of official monitoring.

Further, the German Raw Milk Quality Regulation (Rohmilch Güteverordnung RohmilchGütV) of July 1, 2021, includes requirements for testing to detect inhibitors, such as quinolones, as part of milk quality testing, in particular a sample from each dairy farm has to be tested for quinolones twice per year. According to the RohmilchGütV, a suitable detection method is required to detect at least one of the three substances CIP, ENR or MAR with a detection limit of 100 $\mu$g/kg for CIP and ENR or 75 $\mu$g/kg for MAR.

Analytical methods are used for testing raw milk for inhibitors, among others, immunological and various chemical-physical methods, which allow sensitive and quantitative detection as well as substance identification of fluoroquinolones in milk.

Due to the sample preparation, required technical equipment and qualified personnel, these methods are considered to be complex, time-consuming and cost-intensive.

In addition, microbial test systems based on agar diffusion methods are known.

Agar diffusion methods for testing milk for the presence of inhibitors are often based on the use of *Geobacillus stearothermophilus* as an indicator strain. Spores of the indicator strain are embedded in a nutrient agar, test samples are added and incubated, and the presence or absence of bacterial growth can be used to determine the presence of inhibitors.

The Brilliant Black Reduction Test (BRT) using *Geobacillus stearothermophilus var. calidolactis* C953 as indicator bacteria according to method L 01.01-5 (January 2012) and method L 01.00-11 (December 2002) of the official collection of examination procedures according to § 64 of the German Food, Commodities, and Feed Code (Lebensmittel-, Bedarfsgegenstände- und Futtermittelgesetzbuch LFGB) represents such a method.

This BRT is primarily known for its sensitive detection of inhibitors such as penicillins and can be used to detect antibiotics of other substance classes at the level of the legally required maximum residue levels (MRL). However, inhibitors such as fluoroquinolone antibiotics currently cannot be detected at the level of their respective legally required MRLs (see above, according to RohmilchGütV and Commission Regulation (EU) 37/2010100 - ENR and CIP: 100 $\mu$g/kg for, DAN: 30 $\mu$g/kg, MAR: 75 $\mu$g/kg, FLU 50 $\mu$g/kg). For example, detection of enrofloxacin with *Geobacillus stearothermophilus-based* BRT assays has been reported at concentrations in the range from 1000 to 4000 $\mu$g/kg, whereas the MRL for enrofloxacin in milk is 100 $\mu$g/kg, according to Commission Regulation (EU) 37/2010. Therefore, there is a need for a reliable, fast, simple and cost-efficient way of detecting inhibitors in milk, at or even below their legally required MRLs.

Muhammad and Ahmed, "Production and characterization of a new antibacterial peptide obtained from Aeribacillus pallidus SAT4", Biotechnology Repoorts, vol. 8: 72-80, 2015, concerns the identification of an antibacterial peptide produced by *Aeribacillus pallidus* SAT4.

Harirchi et al., "Efficacy of polyectremophilic Aeribacillus pallidus on bioprocessing of beet vinasse derived from ethanol industries", Bioresource Technology, vol. 313, 2020, concerns the use of *Aeribacillus pallidus* for the aerobic processing of beet vinasse.

Guna et al., "In vitro activity of linezolid, clarithromycin and moxifloxacin against clinical isolates of Mycobacterium kansasii", Journal of Antimicrobial Chemotherapy, vol. 55: 950-953, 2005, concerns the sensitivity of *Mycobacterium kansasii* to several antibiotics.

Sala et al., "Simple Model for Testing Drugs agains Nonreplicating Mycobacterium tuberculosis", Antimicrobial Agants and Chemotherapy, vol. 54(10): 4150-4158, 2010, concerns a test system for testing the sensitivity of *Mycobacterium tuberculosis* to antibiotics.

FR 2882370 concerns the detection of microorganisms in a liquid by using at least two dyes.

Wu et al., "A Novel Microbiological Method in Microtiter Plates for Screening Seven Kinds of Widely Used Antibiotics Residues in Milk, Chicken Egg and Honey", Front. Microbiol., vol. 10(436): 1-11, 2019, concerns *Geobacillus stearothermophilus var C953* for detecting antibiotics in milk, chicken egg and honey.

## SUMMARY OF THE INVENTION

The present invention is defined in the claims. Embodiments of the present description not falling under the scope of the claims are not part of the present invention.

It is the objective of the present invention to provide a reliable, fast, simple and cost-efficient way of detecting inhibitors in milk, at or even below their legally required MRLs.

This objective is achieved according to the present invention by:
A microbial test system for detecting an inhibitor of bacterial growth in milk comprising indicator bacteria of the species *Aeribacillus pallidus* embedded in a growth medium, wherein the growth medium contains a dye capable of changing color upon growth of the indicator bacteria in the growth medium, and wherein the growth medium contains 8-hydroxyquinoline.

In another aspect, the present invention relates to a use of indicator bacteria of the species *Aeribacillus pallidus* for detecting an inhibitor of bacterial growth in milk.

In another aspect, the present invention relates to a method for detecting an inhibitor of bacterial growth in milk comprising the steps of a) contacting a milk sample with indicator bacteria of the species *Aeribacillus pallidus,* wherein the indicator bacteria are embedded in a growth medium, b) incubating the indicator bacteria in the growth medium under conditions suitable for growth of the indicator bacteria, and c) determining the growth of the indicator bacteria in the growth medium, wherein the inhibitor is present in the milk sample if the growth of the bacteria is inhibited.

In another aspect, the present invention relates to a test kit for detecting an inhibitor of bacterial growth in milk comprising indicator bacteria of the species *Aeribacillus pallidus* and a growth medium, wherein the growth medium contains a dye capable of changing color upon growth of the indicator bacteria in the growth medium, and wherein the growth medium further contains 8-hydroxyquinoline.

In another aspect, the present invention relates to a growth medium for detecting an inhibitor of bacterial growth in milk comprising a dye capable of changing color upon growth of an indicator bacteria in the growth medium, preferably brilliant black BN, and moxifloxacin and 8-hydroxyquinoline.

## BRIEF DESCRIPTION OF DRAWINGS

**Figure 1.** Comparison of the spore production on different sporulation media. The strains were cultivated on different solid sporulation media (tryptone soy agar (TSA), milk agar, skimmed milk agar, and meat extract agar), and a comparison of the spore amount was used to investigate the extent to which sporulation is dependent on the sporulation medium used.

**Figure 2.** Strain-dependent optimized conditions for spore production. Sporulation rates after incubation of strain cultures for 24, 48 and 72 h were determined.

**Figure 3.** Sensitivity of different bacterial strains. Sensitivity of different bacterial strains to inhibitors was determined with a disk diffusion test ("Blättchentest"), in which diameters of the inhibition zone were determined for fluoroquinolones enrofloxacin (ENR), norfloxacin (NOR) and moxifloxacin (MOX).

**Figure 4.** Sensitivity of different bacterial strains. Sensitivity of different bacterial strains to inhibitors was determined for inhibitors enrofloxacin (ENR), danofloxacin (DAN), levofloxacin (LEV), marbofloxacin (MAR), moxifloxacin (MOX) and norfloxacin (NOR) in the microdilution test (MD) and for ciprofloxacin (CIP), ENR, MAR and NOR in the gradient diffusion test (GD).

**Figure 5.** Determination of incubation times of test systems. The effect of different pH values of the growth media on corresponding incubation times until complete discoloration of the negative control was achieved.

**Figure 6.** Detection sensitivities of test systems. The detection sensitivity of manufactured BRT systems for addition of different concentrations of different gyrase inhibitors was evaluated in relation to the MRLs.

**Figure 7.** Germination and growth curves of strains *G. stearothermophilus* C953 (DSM 1550) and A. *pallidus* MHI 3391 with and without Ca-DPA activation in MH broth at pH $8.5 \pm 0.1$. Extinction values of non-treated spores were compared with extinction values of spores after Ca-DPA activation.

**Figure 8.** Dose-response curves for (8A) CIP, (8B) ENR, (8C) DAN and (8D) MAR. Depicted are quality curves, as a result of photometric evaluation.

**Figure 9.** Detection limits of the FQ-BRT system for different inhibitors depending on different detection methods (photometric and visual). Detection limits (CCβ) of the FQ-BRT for inhibitors used for therapeutic purposes in veterinary and human medicine were calculated using a logistic regression model.

**Figure 10.** Discoloration time of a modified BRT system with *A. pallidus* MHI 3391 after incubation at different temperatures. The effect of various incubation temperatures on the growth and the brilliant black reduction of the indicator strain *A. pallidus* MHI 3391 was determined.

**Figure 11.** Detection of inhibitors belonging to various antibiotic classes. The detection of inhibitors belonging to various antibiotic classes with the test system according to the disclosure has been evaluated.

## DETAILED DESCRIPTION OF THE INVENTION

Unless defined otherwise, all technical and scientific terms used herein have the meaning commonly understood by a person skilled in the art to which this invention belongs.

The use of the term "comprising" as well as other grammatical forms such as "comprises" and "comprised" is not limiting. The terms "comprising", "comprises" and "comprised" should be understood as referring to an open-ended description of an embodiment of the present disclosure that may, but does not have to, include additional technical features in addition to the explicitly stated technical features.

In the same sense the term "involving" as well as other respective grammatical forms such as "involves" and "involved" is not limiting. The same applies for the term "including" and other grammatical forms such as "includes" and "included". Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

Section headings throughout the description are for organizational purposes only. In particular, they are not intended as limiting for the embodiments described therein, and it is to be understood that embodiments (and features therein) described under one subheading may be freely combined with embodiments (and features therein) described under another subheading.

As used herein, "a" or "an" may mean one or more. As used herein when used in conjunction with the word "comprising," the words "a" or "an" may mean one or more than one. As used herein "another" may mean at least a second or more. Furthermore, unless otherwise required by context, singular terms include pluralities and plural terms include the singular.

As used herein, "about" refers to a numeric value, including, for example, whole numbers, fractions, and percentages, whether or not explicitly indicated. The term "about" generally refers to a range of numerical values that one of ordinary skill in the art would consider equivalent to the recited value (e.g., having the same function or result).

In some instances, the term "about" may include numerical values that are rounded to the to the last decimal place.

As used herein, the term "microbial test system" or any other grammatical form of this term may be used interchangeably with the term "test system". In some embodiments, if the microbial test system comprises the dye Brilliant Black, the test system may be designated as a "BRT system" or "BRT test system".

As used herein, the term "inhibitor" any other grammatical form of this term refers to a substance that leads to growth inhibition of bacteria. The inhibitor may be an antibiotic, in particular a quinolone antibiotic, such as a fluoroquinolone.

As used herein, the term "indicator bacteria" or any other grammatical form of this term may be used interchangeably with the term "bacteria".

The term indicator bacteria may refer to thermophilic, spore-forming bacteria, preferably from the families *Bacillaceae* and *Paenibacillaceae,* more preferably of the genus *Aeribacillaceae,* such as *Aeribacillus pallidus.* As used herein, the term *"Aeribacillus pallidus"* may be used interchangeably with the term *"A. pallidus".*

As used herein, the term "spore suspension" or any other grammatical form of this term may be used interchangeably with the terms "spore" and "endospore". The term "spore" refers to a resistant permanent form of bacteria, which allows persistence even in the absence of necessary growth conditions.

As used herein, the term "milk" refers to a white, cloudy emulsion or colloidal dispersion of proteins, lactose and milk fat in water produced by the mammary glands of mammals, such as dairy cattle, goats, sheep, donkey, camels, yaks, elk, water buffalo, and horses.

## I. Microbial test system

A first aspect of the disclosure relates to microbial test system for detecting inhibitors in milk comprising indicator bacteria of the species *Aeribacillus pallidus* embedded in a growth medium. In a preferred embodiment the embedded indicator bacteria are spores, more preferably endospores.

In a more preferred embodiment, the indicator bacteria are *Aeribacillus pallidus* MHI 3391 or *Aeribacillus pallidus* MHI 3390. Particularly preferred is *Aeribacillus pallidus* MHI 3391.

In a preferred embodiment, the microbial test system enables detection of inhibitors in milk at or below their legally required MRLs, for instance of 100 µg/kg for ENR and CIP, 30 µg/kg for DAN, 75 µg/kg for MAR, and 50 µg/kg for FLU. In a more preferred embodiment, the microbial test system enables detection of inhibitors in milk below their legally required MRLs of 100 µg/kg for ENR and CIP, and 75 µg/kg for MAR.

The growth medium comprises a dye capable of changing color upon growth of the indicator bacteria in the growth medium. In particular, the dye changes its color if the bacteria grow and the dye does not change its color if the bacteria do not grow, e.g. because their growth is inhibited by the presence of an inhibitor, such as an antibiotic. Growth or absence of growth of bacteria, and therefore absence or presence, respectively, of inhibitor, can therefore be assessed based on the color of the dye.

In some embodiments, the dye is an azo dye, a diazo dye, a triazo dye, a tetrakisazo dye, a polyazo dye, a triarylmethane dye, an anthraquinone dye or a dioxazine dye. In a preferred embodiment, the dye is a diazo dye. In a more preferred embodiment, the dye is tetrasodium (6Z)-4-acetamido-5-oxo-6-[[7-sulfonato-4-(4-sulfonatophenyl)azo-1-naphthyl]hydrazono]naphthalene-1,7-disulfonate, also known as brilliant black BN, CI Food Black 1, 1743 Black, Black PN, Blue Black BN, Brilliant Acid Black, CI 28440, Certicol Black PNW, Cilefa Black B, E 151, Edicol Supra Black BN, Hexacol Black PN, L Black 8000, Melan Black, or Xylene Black F, which can be used interchangeably. In another preferred embodiment, the dye is a triarylmethane dye, preferably bromocresol purple.

In some embodiments, the dye is present in the growth medium at a concentration of about 50-200 mg/l, for instance at about 50, 55, 60, 65, 70, 80, 85, 90, 95, 100, 105, 110, 115, 120, 125, 130, 135, 140, 145, 150, 155, 160, 165, 170, 175, 180, 185, 190, 195, or 200 mg/l, preferably at a concentration of about 80-120 mg/l, more preferably at a concentration of about 100 mg/l.

The addition of sub-inhibitory concentrations of antibacterial agents, such as fluoroquinolone, to the growth medium increases the sensitivity to the inhibitor to be detected. In some embodiments, the growth medium comprises ciprofloxacin, besifloxacin, delafloxacin, levofloxacin, sodium cholate, polysorbat-20, nisin and/or inhibitors of multi drug resistance pumps, such as carbonylcyanide-m-chlorphenylhydrazone (CCCP), reserpine, verapamil or thioridazine. In particular, the growth medium may comprise ciprofloxacin. In a preferred embodiment, the growth medium contains moxifloxacin and 8-hydroxyquinoline. In a more preferred embodiment, the growth medium contains moxifloxacin at about 100 μg/l and 8-hydroxyquinoline at about 8000 μg/l. The additives listed above result in an increase of detection sensitivity and/or may reduce the required incubation time. In the present invention, the addition of 8-hydroxyquinoline reduces the required incubation time.

In some embodiments, the growth medium has a pH value of about pH 8.5.

In some embodiments, the growth medium is a growth medium as defined elsewhere herein.

A further aspect of the invention relates to the use of indicator bacteria of the species *Aeribacillus pallidus* as taught herein for detecting an inhibitor in milk.

Another aspect of the invention relates to a method for detecting an inhibitor in milk comprising the steps of:

a) contacting a milk sample with indicator bacteria of the species *Aeribacillus pallidus* embedded in a growth medium,

b) incubating the indicator bacteria in the growth medium under conditions suitable for growth of the indicator bacteria, and

c) determining the growth of the indicator bacteria in the growth medium,
wherein the inhibitor is present in the milk sample if the growth of the bacteria is inhibited.

In some embodiments, the indicator bacteria are comprised in a microbial test system as described herein.

In some embodiments, in step b) the indicator bacteria are incubated at about 55°C to about 70°C, for instance at about 55°C, 56°C, 57°C, 58°C, 59°C, 60°C, 61°C, 62°C, 63°C, 64°C, 65°C, 66°C, 67°C, 68°C, 69°C, or 70°C, preferably at about 60°C to about 62°C, more preferably at about 61°C to ensure optimal growth conditions for germinated spores/vegetative cells.

In some embodiments, the indicator bacteria are *Aeribacillus pallidus* MHI 3391, *Aeribacillus pallidus* MHI 3390.

The term *"Aeribacillus pallidus"* may be used interchangeably with the term *"A. pallidus"*.

In a further aspect of the invention, a test kit for detecting an inhibitor in milk comprising indicator bacteria of the species *Aeribacillus pallidus* is provided, as defined in the claims.

## II. Indicator bacteria and spore suspensions

In some embodiments, the indicator bacteria are thermophilic, aerobe, spore-forming bacteria. In some embodiments, the indicator bacteria are of the families *Bacillaceae* and *Paenibacillaceae,* preferably of *Aeribacillaceae*. In a preferred embodiment, the indicator bacteria are of the species *Aeribacillus pallidus.* In a more preferred embodiment, the indicator bacteria are *Aeribacillus pallidus* MHI 3391.

In certain embodiments, the *Aeribacillus pallidus* strain for use in accordance with the microbial test system, the method, the test kit or the growth medium described herein can be selected from the group consisting of *Aeribacillus pallidus* MHI 3391 (DSM 34046) *and Aeribacillus pallidus* MHI 3390.

*Aeribacillus pallidus* MHI 3391 has been deposited on October 1, 2021 at the German Collection of Microorganisms and Cell Cultures GmbH (DSMZ), Germany, 38124 Braunschweig, and was given accession number DSM 34046. The deposit has been made by Ludwig-Maximilians-Universität, Department of Hygiene and Technology of Milk, Schönleutnerstraße 8, 85764 Oberschleißheim.

The depositor has authorized the applicant to refer to the deposited biological material in the present application and has given his unreserved and irrevocable consent to the deposited material being made available to the public in accordance with Rule 33 EPC (Rule 31(1)(d) EPC).

Spores are a resistant permanent form of bacteria, which allows persistence even in the absence of necessary growth conditions. Spores are thus stable to environmental conditions and germinate only after incubation at higher temperatures (about 55°C or higher), i.e. have to be activated by heat for germination. After germination of the spores, the vegetative cells multiply and quickly reach their log phase due to their short generation time. Bacteria in this growth phase are optimal for the microbial test system described herein. Bacteria in this growth phase are constantly dividing, have an active metabolism, and their cell wall are in permanent formation and degradation. This ensures fast absorption of substances.

The spores are stored at low temperatures, which interrupts germination and multiplication. Thus, storage of the product is possible and heat activation before using the microbial test system is unnecessary.

Extrinsic factors during the sporulation process affect the extent of sporulation and the individual properties of spores. For example, thermophilic species form spores exclusively at temperatures within their growth range. Temperature, pH and the presence of nutrients and ions are further factors influencing sporulation properties. It was found that the use of endospore-forming indicator strains with short generation times enables test systems with incubation times of only a few hours. Accordingly, in some embodiments the indicator bacteria are endospore-forming.

In some embodiments, the incubation time of incubating the indicator bacteria is in the range of one to six hours, i.e. 60 min to 360 min, for instance at 60, 75, 90, 105, 120, 135, 150, 165, 180, 195, 210, 225, 240, 255, 270 min, 285, 300 min, 315, 330, 345 or 360 min.

In some embodiments, the incubation time of incubating the indicator bacteria of the species *Aeribacillus pallidus* is reduced in comparison to the incubation time of incubating indicator bacteria of the species G. *stearothermophilus* C953 (DSM 1550) in the range of one to six hours, i.e. 60 min to 360 min, for instance at 60, 75, 90, 105, 120, 135, 150, 165, 180, 195, 210, 225, 240, 255, 270 min, 285, 300 min, 315, 330, 345 or 360 min. in a preferred embodiment, the incubation time is reduced by 3 hours, in a more preferred embodiment the incubation time is reduced by 2 hours.

In some embodiments, the indicator bacteria are present in the form of spores. In an embodiment, the indicator bacteria are present in the form of endospores. In a preferred embodiment, the indicator bacteria of the species *Aeribacillus pallidus* are present in the form of spores. In a more preferred embodiment, the indicator bacteria of the species *Aeribacillus pallidus* are present in the form of endospores.

In a further aspect, the disclosure relates to the use of indicator bacteria for detecting an inhibitor in milk. In some embodiments, the indicator bacteria are comprised in a microbial test system as described herein.

Preferably the indicator bacteria of the present disclosure are spores.

In some embodiments, the spore suspension is treated after spore harvest, preferably at about 80°C to about 100°C for about 15-30 min, for instance for about 16, 17, 18, 19, 20, 21, 22, 23, 24, 25,26, 27, 28 or 29 min, to inactivate vegetative bacterial cells. Spores may grow while incubating the spores in a growth medium under conditions suitable for growth of the spores, e.g. in the absence of an inhibitor, thereby enabling use as indicator bacteria for detecting an inhibitor in milk in the microbial test system, the method or the test kit of the present disclosure.

Preferably, the spore suspension is treated at about 80°C to about 100°C before embedding the spores in the growth medium, in particular of the microbial test system or the test kit.

In an embodiment, the vegetative cells are heat-inactivated at about 80°C to about 100°C, for instance at about 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, or 100°C, preferably at about 80°C to about 100°C, more preferably at about 90°C.

In some embodiments, the vegetative cells are heat-inactivated for about 15 to about 30 min, for instance at about 15, 16, 17, 18, 19, 20, 21, 22, 23, 24, 25, 26, 27, 28, 29, or 30 min, preferably for about 20 to 25 min, more preferably at about 20 min. In a more preferred embodiment, the spores are heat-activated at about 90 °C for about 20 min.

In some embodiments, the growth medium comprises a number of spores of about $1.5 \times 10^4$ cfu/ml to about $2.5 \times 10^8$ cfu/ml, for instance at about $6.25 \times 10^7$, $1.56 \times 10^7$, $3.91 \times 10^6$, $9.77 \times 10^5$, $2.44 \times 10^5$, $6.10 \times 10^4$, $1.50 \times 10^4$, preferably at about $9.77 \times 10^5$ cfu/ml to about $1.56 \times 10^7$ cfu/ml.

In an embodiment, the indicator bacteria, preferably the spores, are incubated at about 35°C to about 75°C, for instance at about 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C or 75°C. These temperatures are temperatures suitable for growth of the indicator bacteria of the present disclosure. In a preferred embodiment, the indicator bacteria, preferably the spores, are incubated at about 35°C to about 65°C, optionally at about 45°C to about 65°C. In a more preferred embodiment the spores are incubated at about 55°C to about 65°C. Incubation at the mentioned temperature leads to germination of the spores.

In an embodiment, bacterial strains are incubated on solid or liquid media as follows:

Table 1: Sporulation media

| Tryptone soy agar (TSA) | 40 g | TSA |
|---|---|---|
| | 1.66 ml | 3 M sodium pyruvate solution |
| | 1 ml | 1 M Ca(NO$_3$)$_2$ x 4 H$_2$O |
| | 1 ml | 0.1 M MnCl$_2$ x 4 H$_2$O |
| | 1 ml | 1 mM FeSO$_4$ x 7 H$_2$O |
| | Ad. 1 l | aqua dest. |

(continued)

| Tryptone soy agar (TSA) | 40 g | TSA |
|---|---|---|
| Milk agar | 24 g | milk agar |
| | 50 mg | $MnCl_2$ x 4 $H_2O$ |
| | 1.66 ml | 3 M sodium pyruvate solution |
| | Ad. 1 l | aqua dest. |
| Skimmed milk agar | 800 ml initial milk | Milupa Aptamil PronutraPre ready-to-drink |
| | 14.4 g | agar |
| | 1.6 g | ammonium sulfate |
| | 1.66 ml | 3 M sodium pyruvate solution |
| | 960 $\mu l$ | 10 mM $MnCl_2$ x 4 $H_2O$ |
| | 160 ml | aqua dest. |
| Meat extract agar | 20 g | agar |
| | 5 g | peptone |
| | 4 g | yeast extract |
| | 3 g | meat extract |
| | 19.79 mg | $MnCl_2$ x 4 $H_2O$ |
| | 1.66 ml | 3 M sodium pyruvate solution |
| | Ad. 1 l | aqua dest. |

In some embodiments, the microbial test system, the method, the use, the test kit or the growth medium described herein comprises spores of *Aeribacillus pallidus* MHI 3391, *Aeribacillus* MHI 3390 as indicator bacteria, brilliant black BN at a concentration of about 100 mg/l, moxifloxacin at a concentration of about 100 $\mu$g/l and 8-hydroxyquinoline at a concentration of about 8000 $\mu$g/l.

In a preferred embodiment, the microbial test system, the method, the use, the test kit or the growth medium described herein comprises spores of *Aeribacillus pallidus* MHI 3391 as indicator bacteria, brilliant black BN at a concentration of about 100 mg/l, moxifloxacin at a concentration of about 100 $\mu$g/l and 8-hydroxyquinoline at a concentration of about 8000 $\mu$g/l.

In some embodiments, the growth medium is a bacterial growth medium suitable to culture bacteria, preferably *Aeribacillus pallidus* MHI 3391.

In a preferred embodiment, the growth medium comprises about 100 mg/l brilliant black BN, about 100 $\mu$g/l moxifloxacin, about 8000 $\mu$g/l 8-hydroxyquinoline, meat extract at about 1.5 g/l, yeast extract at about 3.0 g/l, casein peptone at about 4.0 g/l, meat peptone at about 6.0 g/l, D(+)-glucose at about 1.0 g/l, trimethoprim solution at about 10 ml/l, agar at about 10 to about 15 g/l and has a pH value of about pH 8.5.

## III. Inhibitors

The inhibitor, in particular to be detected in milk in accordance with the microbial test system, the use, the method, the test kit or the growth medium described herein, is a substance that leads to growth inhibition of bacteria.

In some embodiments, the inhibitor is an antibiotic. In some embodiments, the inhibitor is a quinolone. In a preferred embodiment, the inhibitor is a fluoroquinolone. In a further embodiment, the inhibitor is a quinolone selected from the group consisting of oxolinic acid, norfloxacin, flumequine, ciprofloxacin, enrofloxacin, ofloxacin, nadifloxacin, sarafloxacin, difloxacin, levofloxacin, orbifloxacin, marbofloxacin, danofloxacin, pradofloxacin, moxifloxacin or combinations thereof.

In a more preferred embodiment, the inhibitor is ciprofloxacin, enrofloxacin, marbofloxacin, danofloxacin, or combinations thereof.

In some embodiments, as little as about 100 $\mu$g ciprofloxacin per about 1 kg milk and about 75 $\mu$g enrofloxacin or marbofloxacin per about 1 kg milk can be detected.

In some embodiments, the detection limit for ciprofloxacin is in the range of about 10 to 60 $\mu$g per about 1 kg milk, for enrofloxacin in the range of about 50 to 90 $\mu$g per about 1 kg milk, for danofloxacin in the range of about 60 to about 100 $\mu$g per about 1 kg milk, for marbofloxacin in the range of about 60 to about 80 $\mu$g per about 1 kg milk, for flumequine in the range of about 1280 to about 2050 $\mu$g per about 1 kg milk, and/or for nadifloxacin, levofloxacin, and moxifloxacin in the range of about 5 to 60 $\mu$g per about 1 kg milk. In a preferred embodiment, the detection limit for ciprofloxacin is in the range of about 29,0 to 39,6 $\mu$g per about 1 kg milk, for enrofloxacin in the range of about 56,1 to 83,7 $\mu$g per about 1 kg milk, for nadifloxacin, levofloxacin, and/or moxifloxacin in the range of about 6,7 to 54,4 μg per about 1 kg milk.

According to the Regulation (EU) 37/2010 (Annex, Table 1) the maximum residue levels (MRLs) of enrofloxacin (ENR) and its metabolite ciprofloxacin (CIP) in milk of bovine, ovine and caprine species is limited to 100 μg/kg, 30 μg/kg for danofloxacin (DAN) in milk of bovine, ovine and caprine species, 75 μg/kg for marbofloxacin (MAR) in milk of bovine species, and 50 μg/kg for flumequine (FLU) in milk of bovine, ovine and caprine species. According to RohmilchGütV, a suitable detection method is required to detect at least one of the three substances CIP, ENR or MAR with a detection limit of 100 μg/kg for CIP and ENR or 75 μg/kg for MAR. In some embodiments, the detection limit allows detecting the inhibitor at the maximum residue level as defined in Regulation (EU) 37/2010 and according to RohmilchGütV, or below.

In a preferred embodiment, the indicator bacteria of the species *Aeribacillus pallidus* is *Aeribacillus pallidus* MHI 3391 and the inhibitor in milk to be detected is ciprofloxacin, enrofloxacin, danofloxacin and/or marbofloxacin.

IV. Growth medium

Another aspect of the invention relates to a growth medium for detecting an inhibitor of bacterial growth in milk comprising a dye capable of changing color upon growth of an indicator bacteria in the growth medium and moxifloxacin and 8-hydroxyquinoline.

Furthermore, another aspect of the invention relates to a growth medium suitable for growing thermophilic, spore-forming bacteria. In some embodiments, the bacteria are from the families *Bacillaceae* and *Paenibacillaceae*, preferably of *Aeribacillaceae*. In a preferred embodiment, the bacteria are of the species *Aeribacillus pallidus*. In a more preferred embodiment, the bacteria are *Aeribacillus pallidus* MHI 3391.

In an embodiment, the growth medium is suitable for storing indicator bacteria, for instance at ambient temperature, without refrigeration. In another embodiment, the growth medium is suitable for storing indicator bacteria at a temperature of about 0°C to about -30°C. In a preferred embodiment, the growth medium is suitable for storing indicator bacteria for use in a microbial test system.

In some embodiments, the growth medium is used in a microbial test system as described herein. In an embodiment, the growth medium is for carrying out an agar diffusion assay.

The growth medium comprises a dye selected from the group consisting of an azo dye, a diazo dye, a triazo dye, a tetrakisazo dye, a polyazo dye, a triarylmethane dye, an anthraquinone dye or a dioxazine dye. In a preferred embodiment, the dye is a diazo dye.

In a more preferred embodiment, the dye is brilliant black BN. In another preferred embodiment, the dye is a triarylmethane dye, more preferably Bromocresol purple. In an embodiment, the dye is present in a concentration of 50-200 mg/l, for instance at about 50, 55, 60, 65, 70, 80, 85, 90, 95, 100, 105, 110, 115, 120, 125, 130, 135, 140, 145, 150, 155, 160, 165, 170, 175, 180, 185, 190, 195, or 200 mg/l, preferably in a concentration of about 80-120 mg/l, more preferably in a concentration of about 100 mg/l.

In some embodiments, the growth medium comprises at least one of the following: meat extract, yeast extract, casein peptone, tryptic digested, meat peptone, tryptic digested, D(+)-glucose, trimethoprim solution, and agar.

In a preferred embodiment, the meat extract is present at about 1.5 g/l, the yeast extract at about 3.0 g/l, the casein peptone at about 4.0 g/l, the meat peptone at about 6.0 g/l, the D(+)-glucose at about 1.0 g/l, the trimethoprim solution at about 10 ml/l, and/or the agar at about 10 to about 15 g/l. This growth medium is also referred as BRT basis medium hereinafter.

In a more preferred embodiment, the meat extract is present at about 1.5 g/l, the yeast extract at about 3.0 g/l, the casein peptone at about 4.0 g/l, the meat peptone at about 6.0 g/l, the D(+)-glucose at about 1.0 g/l, the trimethoprim solution at about 10 ml/l, and/or the agar at about 10 to about 15 g/l and the dye capable of changing color upon growth of an indicator bacteria is brilliant black.

In some embodiments, the growth medium has a pH value of about pH 5.5 - pH 9.5. In an embodiment, the growth medium has a pH value of about pH 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3 or 9.4. In a preferred embodiment, the growth medium has a pH value of about pH 8.5.

In some embodiments, the growth medium comprises brilliant black BN, moxifloxacin, 8-hydroxyquinoline, meat extract at about 1.5 g/l, yeast extract at about 3.0 g/l, casein peptone at about 4.0 g/l, meat peptone at about 6.0 g/l, D(+)-glucose at about 1.0 g/l, trimethoprim solution at about 10 ml/l, agar at about 10 to about 15 g/l in 1 l aqua dest. and has a pH value of about pH 8.5.

In some embodiments, moxifloxacin is present at about 50 μg/l - 150 μg/l. In an embodiment, moxifloxacin is present at about 50, 55, 60, 65, 70, 75, 80, 85, 90, 95, 100, 105, 110, 115, 120, 125, 130, 135, 140, 145 or 150 μg/l. In a preferred embodiment, moxiflocacin is present at about 100 - 150 μg/l. The presence of moxifloxacin in the growth medium leads to an increase in detection sensitivity.

In an embodiment, 8-hydroxyquinoline is present at about 250 μg/l - 10000 μg/l. In a preferred embodiment, 8-hydroxyquinoline is present at about 2000, 2250, 2500, 2750, 3000, 3250, 3500, 3750, 4000, 4250, 4500, 4750, 5000, 5250, 5500, 5750, 6000, 6250, 6500, 6750, 7000, 7250, 7500, 7750, 8000, 8250, 8500, 8750, 9000, 9250, 9500, 9750 or 10000 μg/l. In a more preferred embodiment, 8-hydroxyquinoline is present at about 2000 μg/l - 4000 μg/l. In a highly preferred embodiment, moxifloxacin is present at about 100 μg/l and 8-hydroxyquinoline is present at about 8000 μg/l.

Addition of 8-hydroxyquinoline leads to a reduction of incubation time until complete discoloration of the negative control. In particular, increasing 8-hydroxyquinoline concentration leads to a continuous reduction of the incubation time until complete discoloration of the negative control.

In some embodiments, the growth medium further comprises ciprofloxacin, besifloxacin, delafloxacin, levofloxacin, sodium cholate, polysorbat-20, nisin and/or inhibitors of multidrug resistance pumps, such as carbonylcyanide-m-chlorphenylhydrazone (CCCP), reserpine, verapamil or thioridazine.

In some embodiments, ciprofloxacin is present at about 50 μg/l - 200 μg/l, for instance at about 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or 200 μg/l. In a preferred embodiment, ciprofloxacin is present at about 100 μg/l.

In a preferred embodiment, the growth medium comprises brilliant black BN, about 100 μg/l moxifloxacin, about 8000 μg/l 8-hydroxyquinoline, meat extract at about 1.5 g/l, yeast extract at about 3.0 g/l, casein peptone at about 4.0 g/l, meat peptone at about 6.0 g/l, D(+)-glucose at about 1.0 g/l, trimethoprim solution at about 10 ml/l, agar at about 10 to about 15 g/l and has a pH value of about pH 8.5.

In a more preferred embodiment, the growth medium contains brilliant black BN in a concentration of about 40 mg/l, about 100 μg/l moxifloxacin, about 8000 μg/l 8-hydroxyquinoline, meat extract at about 1.5 g/l, yeast extract at about 3.0 g/l, casein peptone at about 4.0 g/l, meat peptone at about 6.0 g/l, D(+)-glucose at about 1.0 g/l, trimethoprim solution at about 10 ml/l, agar at about 10 to about 15 g/l and has a pH value of about pH 8.5.

## EXAMPLES

The disclosure is now described with reference to the following Examples. These Examples are provided for the purpose of illustration only, and the disclosure is not limited to these Examples.

### EXAMPLE 1 - Bacterial strains

Materials & Methods

Thermophile sporulating bacterial strains of the species *Aeribacillus pallidus,* such as, *Aeribacillus pallidus* MHI 3390 *and/or Aeribacillus pallidus* MHI 3391, were grown on solid or in liquid media at 55 °C in an incubator or shaker incubator and 200 rpm (CERTOMAT S II, Sartorius Stedim Biotech GmbH) overnight. Control strains of S. *aureus and B. cereus* species were incubated at 37 °C. Agar plates were incubated in a humid chamber to prevent desiccation.

Tryptone soy agar (TSA) was used as solid culture medium unless otherwise indicated. Liquid cultures were set up in Müller-Hinton (MH) broth or tryptone soy bouillon (TSB).

### EXAMPLE 2 - Spore suspensions

Materials & Methods

*Inoculation and incubation in sporulation medium*

100 ml Brain-Hearth-Glucose-Bouillon (BHI) was supplemented with 1 mg/l vitamin B12, inoculated with a single colony and incubated at 55 °C and 200 rpm (CERTOMAT S II, Sartorius Stedim Biotech GmbH) overnight.

The culture was concentrated by a factor of 3 by centrifugation at 4,000 × g and 4 °C for 10 min. 3 ml of the concentrated inoculation culture was distributed evenly on the sporulation medium. The spore culture was incubated for 24, 48 or 72 h at 59 °C.

*Harvest of spores*

5 ml sterile distilled water was added into a Roux bottle. The bacterial culture was removed from agar with a metal spatula and transferred to a 50 ml centrifuge tube to obtain a spore suspension. The spore suspension was homogenized by vortexing. The spore suspension was concentrated by a factor of 3 by centrifugation at 6,000 × g and 4 °C for 10 min.

*Inactivation of vegetative cells and storage of spore suspension*

The spore suspension was heated for 20 min at 100 °C, cooled to room temperature (RT) while inverted regularly to prevent the formation of spore agglomerates. The spore suspension was stored at 4 °C.

*Standardization*

For the comparability of measurement results with different microbiological methods, bacterial suspensions were prepared with a standardized plate count. The adjustment to a uniform turbidity level was made using the McFarland standard reference variable or a defined optical density (OD) value at a wavelength of 600 nm.

*Determination of spore count*

Decimal dilution series of the spore suspension were prepared in quarter-strength Ringer's solution. To measure the number of CFU, bacteria were quantified by plating 10-fold serial dilutions (at least two replicates) and colony counting on TSA plates. These plates were incubated for 18 h at 55 °C.

For colony counting, plates with 3 to 300 colonies and morphologically uniform and clearly distinguishable individual colonies were used for evaluation. The spore count of a spore suspension [cfu/ml] was calculated according to the principle of the weighted arithmetic mean.

*Determination of the sporulation rate*

Colony material was removed from the sporulation agar with an inoculation loop and stirred into 15 μl of PBS on a slide. The proportion of spores was visually determined in relation to vegetative cells under a transmitted light microscope at 100 x magnification. The determination was performed in 10 % increments.

### Production and quantification of spore suspensions

In order to determine the extent of media-dependent sporulation, *G. stearothermophilus* C953, *G. stearothermophilus* MHI 3387, *A. pallidus* MHI 3390 and *A. pallidus* MHI 3391 were cultivated on TSA, milk agar, skim milk agar and meat extract agar respectively. The resulting spore yield was compared.

Preliminary experiments revealed a growth-promoting effect of sodium pyruvate for the majority of the selected strains (data not shown). Accordingly, sporulation medium was supplemented with 10 mM sodium pyruvate. The extent of sporulation was quantified based on the sporulation rates of the spore cultures as well as the spore numbers of the prepared spore suspensions.

Prior to spore count determination, the spore suspensions were heated at 100 °C for 10 min to kill vegetative cells and to induce spore germination.

The heating step for *A. flavithermus* MHI 3392 suspensions was performed at 80 °C for 30 min due to a reduced thermoresistance of endospores of this strain.

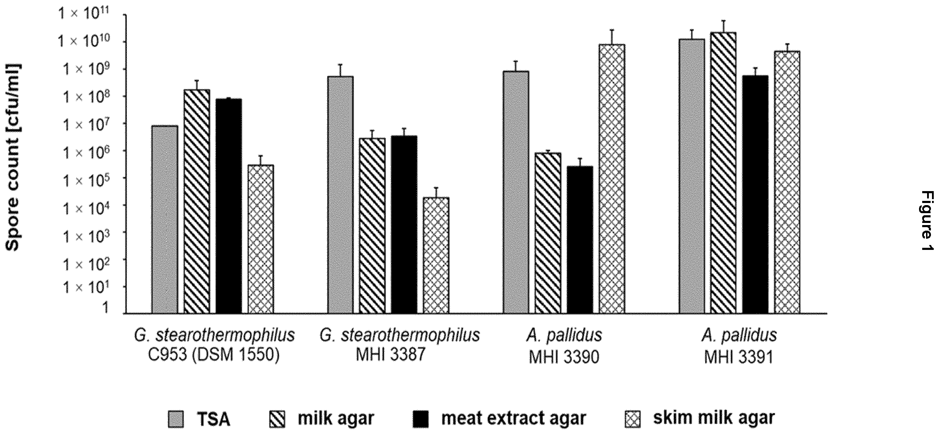

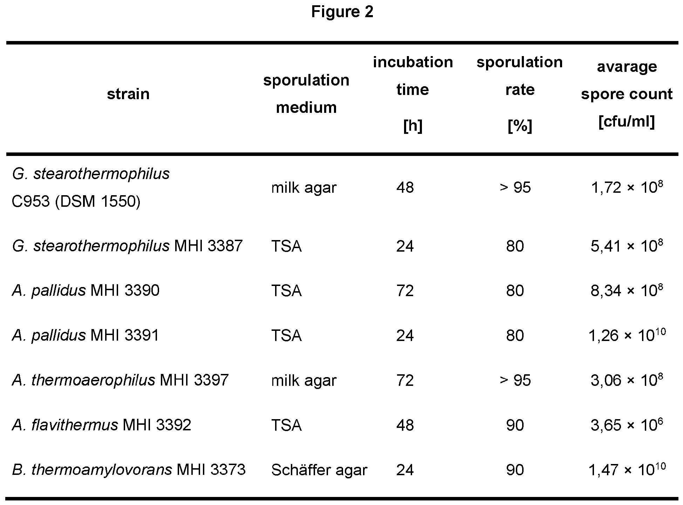

The determination of sporulation rates after incubation of spore cultures for 24, 48 and 72 h showed that an increase in the relative spore content was achieved with increasing incubation time for individual strains. The results are shown in Figure 1 and 2.

### EXAMPLE 3 - Inhibitor sensitivity

### Materials & Methods

*Disk diffusion test ("Blättchendiffusionstest")*

An overnight culture was prepared in MH broth as described herein above (Inoculation and incubation of sporulation media) and a bacterial suspension with a turbidity index corresponding to McFarland = 0.5 was prepared as described herein above (Harvest of spores). 100 μl of the bacterial suspension was plated on MH agar plates. Inhibitor test strips were placed onto agar plates and were pressed down lightly. Agar plates were incubated for 18 h at 55 °C.

The inhibition zone diameter according to EUCAST guidelines (EUCAST, European Committee on Antimicrobial Susceptibility Testing, 2021b) have been determined. *S. aureus* DSM 1104 was used as control strain and compared with CLSI reference values (CLSI, Clinical and Laboratory Standards Institute, 2011).

### Determination of inhibitor sensitivity of bacterial strains

The sensitivity of the bacterial strains *G. stearothermophilus* C953 (DSM 1550), G. *stearothermophilus* MHI 3387, *A. pallidus* MHI 3390 and *A. pallidus* MHI 3391 to ciprofloxacin (CIP), enrofloxacin (ENR), moxiflocacin (MOX) and norfloxacin (NOR) was evaluated using a disk diffusion test.

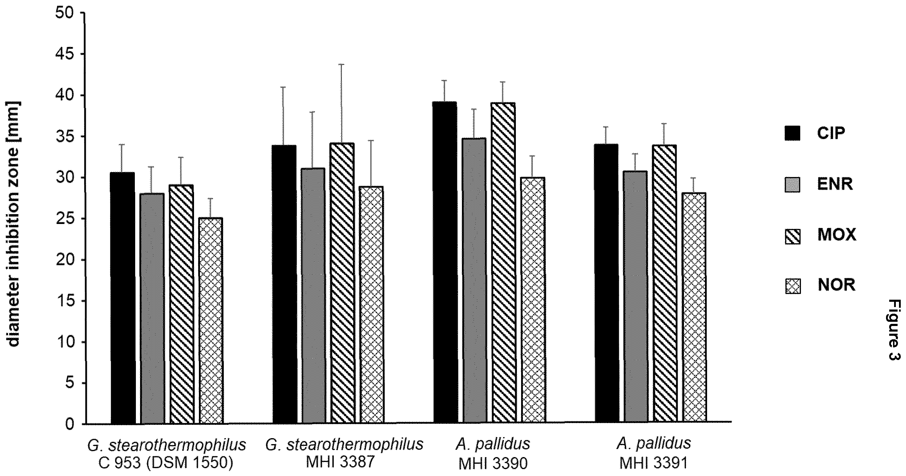

Inhibition zones of 23 to 48 mm of each tested substance were observed in all selected strains. The results are shown in Figure 3.

These results were compared with reference values for *Bacillus* isolates available in clinical microbiology for CIP and NOR (EUCAST, European Committee on Antimicrobial Susceptibility Testing, 2021a), fluoroquinolone resistance was thereby excluded for all strains.

**EXAMPLE 4** - **Detection sensitivity and detection limits**

**Materials & Methods**

*Gradient diffusion test*

A supernatant was prepared in MH broth as described herein above (Inoculation and incubation of sporulation media) and a bacterial suspension with a turbidity index corresponding to McFarland = 0.5 was prepared as described herein above (Harvest of spores). 100 $\mu$l of the bacterial suspension were plated on MH agar plates. Inhibitor test strips were placed on agar plates and were pressed down lightly. Agar plates were incubated for 18 h at 55 °C. The minimum inhibitory concentration (MIC) was determined from the test strip scale according to manufacturer's instructions.

*Microdilution test*

A supernatant was prepared in MH broth as described herein above (Inoculation and incubation of sporulation media) and a bacterial suspension comprising a test strain with a turbidity index corresponding to McFarland = 0.5 was prepared as described herein above (Harvest of spores). 50 $\mu$l of the overnight culture was diluted in 4950 $\mu$l PBS (1st dilution) and vortexed. 500 $\mu$l of the 1st dilution were added to 4500 $\mu$l PBS (2nd dilution) and vortexed. 100 $\mu$l of MH broth was given to each well of a 96-well microtiter plate. Dilution series with a dilution factor of 2 and a total of 10 dilution levels of the substance to be tested were prepared. Each well was inoculated with 5 $\mu$l of the bacterial suspension (2nd dilution). Each bacterial suspension comprising a test strain was tested at least in duplicate.

As reference value and control, sterile medium was used as blank, and inoculated medium was used as control.

$OD_{620}$ was measured at time t = 0 h in the microplate reader. The microtiter plates were incubated at 55 °C for 18 h and $OD_{620}$ was measured again at time t = 18 h. The MIC was determined based on the difference of $OD_{620}$ before and after incubation and subtraction of corresponding blank values ($\Delta OD_{620}$):

$$\Delta OD_{620} = (OD_{620}(t = 18h) - OD_{620}(Ref)) - (OD_{620}(t = 0h) - OD_{620}(Ref))$$

A difference of $\Delta OD_{620} \geq 0{,}01$ was considered as bacterial growth.

**Determination of detection sensitivity and detection limits of different strains**

The sensitivity of *G. stearothermophilus* C953 (DSM 1550), *G. stearothermophilus* MHI 3387 and *A. pallidus* MHI 3391 to ciprofloxacin (CIP), enrofloxacin (ENR), danofloxacin (DAN), marbofloxacin (MAR), Norfloxacin (NOR), Levofloxacin (LEV), and moxifloxacin (MOX) was evaluated using the microdilution test. The sensitivity to CIP, ENR, MAR and NOR was determined using the gradient diffusion test.

In addition, microdilution tests were performed for inhibitors belonging to the group of penicillin, cephalosporins, tetracyclines, sulfonamides, lincosamides, macrolides, or aminoglycoside antibiotics.

Based on the results of the MIC determination of microdilution tests, all strains were sensitive to the inhibitors tested. This also applied to inhibitors of other substance classes. Reference values from clinical microbiology for *Bacillus* spp. were used for this assessment (CLSI, Clinical and Laboratory Standards Institute, 2011).

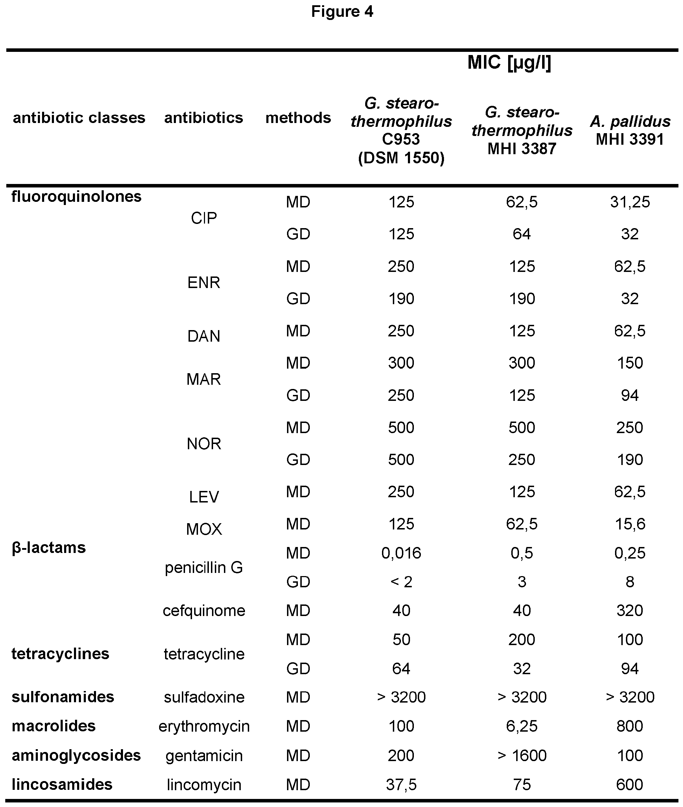

The results of the MIC determination of gradient diffusion tests are comparable with concentration values determined by the microdilution test for all substances. Figure 4 provides an overview of MIC values of inhibitors of different substance classes.

**EXAMPLE 5 - Optimization of growth medium**

*pH value*

Using growth medium with pH values of about 5.5, 6.5, 7.5, 8.5 and 9.5 respectively, the influence on incubation period and inhibitor detection sensitivity of BRT systems was evaluated. Corresponding test systems were prepared as described herein (production of a microbial test system) with a spore count of $1.56 \times 10^7$ cfu/ml of the indicator strains *G. stearothermophilus* C953 (DSM 1550) or *A. pallidus* MHI 3391 respectively.

Both test systems showed a continuous reduction of incubation time until complete discoloration of the negative control on test plates when the pH values were increased. For test plates with *G. stearothermophilus* C953 (DSM 1550) and a pH of 5.5, complete discoloration of the negative control was observed after five hours and 40 minutes. Using pH 9.5, complete discoloration of the negative control was observed after three hours. Similarly, for test plates containing *A. pallidus* MHI 3391, the incubation time was reduced by 2 hours. For plates, which were incubated at pH 5.5, complete discoloration of the negative control was observed after four hours. At pH 9.5 complete discoloration of the negative control was observed after two hours.

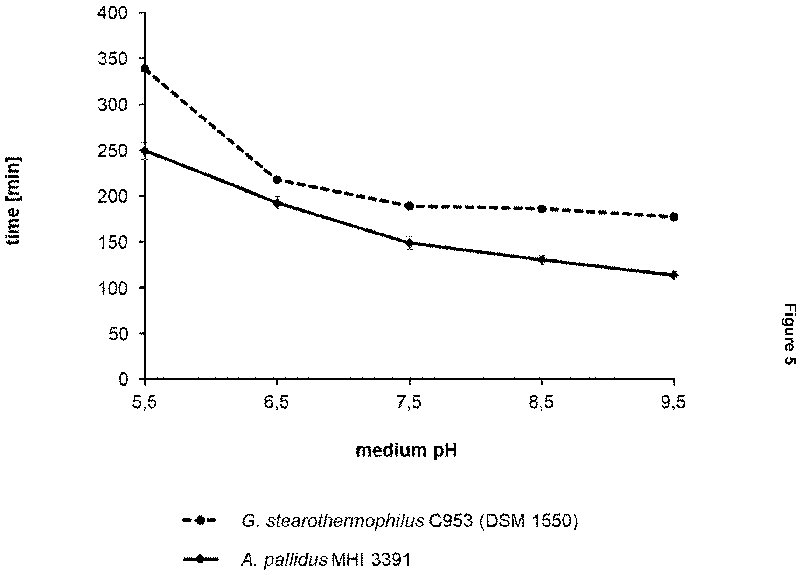

The results are shown in Figure 5. Deviations of detection sensitivities to inhibitors CIP, ENR, DAN and MAR by use of different pH values could not be detected (not shown).

*Addition of gyrase inhibitors*

In order to increase the sensitivity of the microbial test systems to inhibitors, addition of the fluoroquinolones ciprofloxacin (CIP), moxifloxacin (MOX), besifloxacin (BES), delafloxacin (DEL), levofloxacin (LEV) and the aminocoumarin novobiocin, which belongs to the gyrase inhibitors, to the growth medium was evaluated in combination with spore suspensions of *G. stearothermophilus* C953 (DSM 1550) and *A. pallidus* MHI 3391. The medium was prepared with one additive each. The evaluation of an increase in sensitivity and incubation time until complete discoloration of the negative control was performed by comparison with corresponding control medium, spore suspensions from the same batches and an equivalent number of spores.

A concentration-dependent increase in detection sensitivities in microbial test systems with *G. stearothermophilus* C953 (DSM 1550) was observed with growth medium comprising CIP, MOX and LEV. Addition of 50 $\mu$g/l CIP, 75 $\mu$g/l MOX and 100 $\mu$g/l LEV was not associated with an increased sensitivity. Detection of 200 $\mu$g/kg CIP in spiked milk samples was observed with microbial test systems comprising 100 $\mu$g/l CIP, 200 $\mu$g/l CIP, 100 $\mu$g/l MOX, 125 $\mu$g/l MOX, 150 $\mu$g/l MOX, and 200 $\mu$g/l LEV. Improved detection of ENR and MAR was observed with a growth medium comprising 200 $\mu$g/l CIP. Increased sensitivity towards ENR, DAN and MAR was shown by addition of 150 $\mu$g/l MOX.

Improved detection of inhibitors was achieved in microbial test systems by addition of 100 $\mu$g/l CIP. CIP was also detected at concentrations of 100 $\mu$g/kg. Comparable CIP detection was also achieved using growth medium with addition of 50 $\mu$g/l or 75 $\mu$g/l MOX, respectively.

Sensitive and MRL-equivalent detection of CIP, ENR and MAR was achieved by increasing the MOX concentration in the growth medium to 100 $\mu$g/l. Concentrations of 50 $\mu$g/kg CIP, 100 $\mu$g/kg ENR, 120 $\mu$g/kg DAN and 75 $\mu$g/kg MAR were detected in spiked milk samples.

A long incubation period of the test plates was shown by further increasing the MOX concentration to 125 $\mu$g/l and higher. Discoloration of the negative control was detected after more than eight hours.

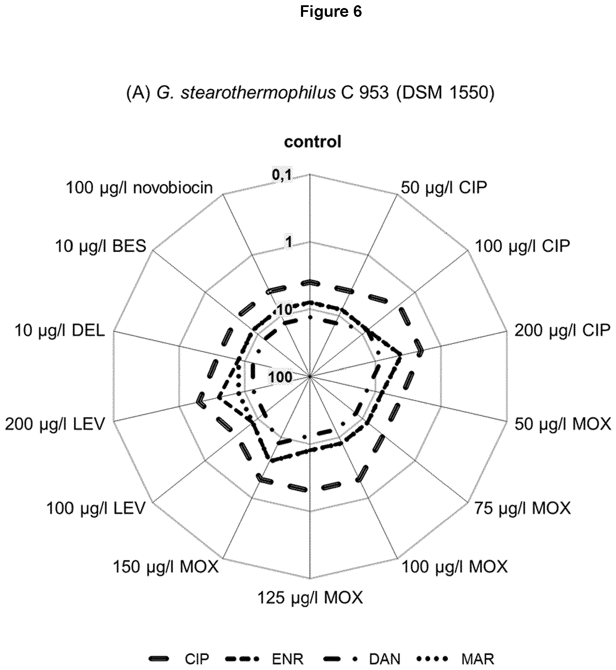

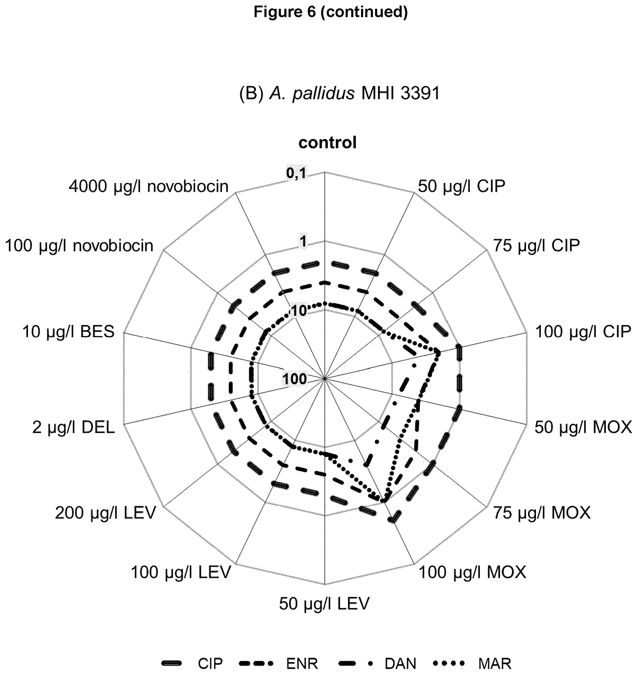

The results are shown in Figure 6.

A longer incubation period of microbial test systems with growth medium comprising gyrase inhibitors are observed compared with corresponding control plates depending on the indicator strains and the concentrations of the gyrase inhibitors used. Test plates with G. *stearothermophilus* C953 (DSM 1550) and a test media addition of 200 $\mu$g/l CIP showed a 32 $\pm$ 10 min longer incubation time until complete discoloration of the negative control, test plates with the same indicator strain and 150 $\mu$g/l MOX showed a 18 $\pm$ 0 min longer incubation time.

BRT systems with *A. pallidus* MHI 3391 and a test media addition of 100 $\mu$g/l CIP were incubated on average 17 $\pm$ 7 min longer than corresponding control plates. The combination of *A. pallidus* MHI 3391 and growth medium comprising 100 $\mu$g/l MOX showed a difference of 62 $\pm$ 31 min.

*Addition of 8-hydroxyquinoline*

The determination of sensitivity of strains *G. stearothermophilus* C953 (DSM 1550) and *A. pallidus* MHI 3391 to 8-hydroxyquinoline resulted in MICs of 4000 and 8000 $\mu$g/l (data not shown).

8-hydroxyquinoline was added at concentrations of 250, 500, 1000, 2000, and 4000 $\mu$g/l to the basic growth medium in test systems comprising *G. stearothermophilus* C953 (DSM 1550) and *A. pallidus* MHI 3391. While no improvement in detection sensitivity was observed in any of the microbial test systems prepared, a continuous reduction in the incubation time until complete discoloration of the negative control was observed in the test systems containing *A. pallidus* MHI 3391 and increasing 8-hydroxyquinoline concentration. Incubation times were reduced by 66 $\pm$ 0 min and 94 $\pm$ 7 min after addition of 2000 or 4000 $\mu$g/l 8-hydroxyquinoline, respectively.

As described herein above, addition of 100 $\mu$g/l MOX or 100 $\mu$g/l CIP to the growth medium was associated with improved fluoroquinolone detection accompanied by an increase in incubation time. Addition of 2000, 4000 and 8000 $\mu$g/l 8-hydroxyquinoline to test systems comprising 100 $\mu$g/l CIP and 100 $\mu$g/l MOX, respectively, and *A. pallidus* MHI 3391 resulted in a reduced incubation time until complete discoloration of the negative control. The addition of 8000 μg/l 8-hydroxyquinoline to growth medium comprising 100 μg/l MOX resulted in an incubation time reduced by $70 \pm 16$ min. With values of $17 \pm 4$ min, the decrease in incubation time was, on average, lower with the combination of 8000 μg/l 8-hydroxyquinoline and 100 μg/l CIP. A reduction in incubation time was not observed in test systems comprising *G. stearothermophilus* C953 (DSM 1550) by addition of 8-hydroxyquinoline.

## EXAMPLE 6 - CA-DPA activated spore suspensions

### Materials & Methods

*Spore activation by means of calcium dipicolinic acid (Ca-DPA)*

120 mM DPA stock solution was prepared in Tris-base and diluted in a 20-80 mM CaCl$_2$ solution to obtain a Ca-DPA working solution before use. A spore suspension was adjusted to a specific spore count of $1.56 \times 10^7$ cfu/ml and a Ca-DPA concentration of 40 mM with Ca-DPA working solution and Ringer's solution. The spore suspension was incubated for 3 h in a thermal shaker at 55 °C and 350 rpm.

### Use of CA-DPA activated spore suspensions

Exogenous addition of calcium dipicolinic acid (Ca-DPA) can artificially initiate spore germination in *Bacillaceae* by activating the peptidoglycan layer-degrading enzyme CwlJ.

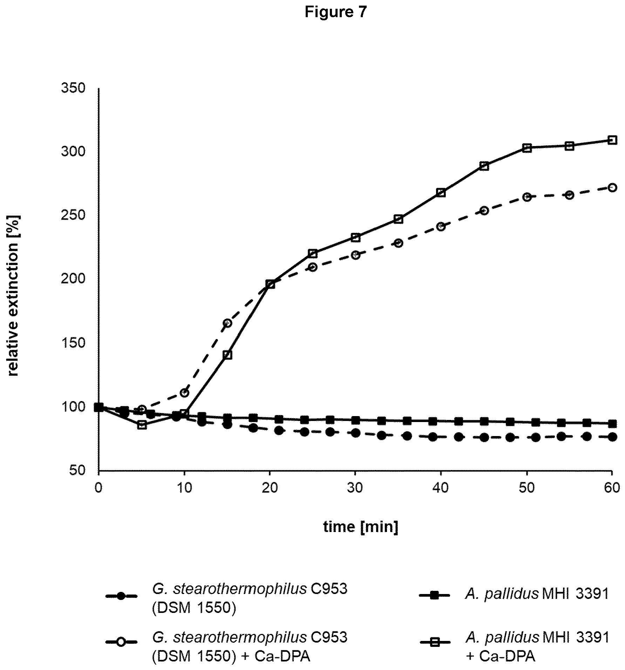

The germination and growth behavior of Ca-DPA-mediated spore activation was evaluated for strains *G. stearothermophilus* C953 (DSM 1550) and *A. pallidus* MHI 3391 in a modified germination and growth assay in MH broth at pH $8.5 \pm 0.1$. Activation of endospores by exogenous Ca-DPA addition was shown: In comparison with the absorbance curves of non-treated spores of these strains, a significantly lower absorbance decrease and an earlier and higher absorbance increase was observed after Ca-DPA activation. Corresponding extraction curves are shown in Figure 7.

The suitability of Ca-DPA-activated spore suspensions in BRT systems was investigated with regard to a reduction of incubation time in comparison with control plates having identical dates of manufacture and testing, but do not comprise Ca-DPA-activated spores.

In test systems with Ca-DPA-activated spore suspensions of *B. thermoamylovorans* MHI 3373, a shorter incubation time of $23 \pm 2$ min was observed. No effect on incubation duration was observed for BRTs with Ca-DPA-activated spore suspensions of *A. thermoaerophilus* MHI 3397.

However, a significant increase in incubation time was observed in test systems with strains *G. stearothermophilus* C953 (DSM 1550), *A. pallidus* MHI 3390, and *A. pallidus* MHI 3391 resulting in a mean difference of $139 \pm 42$ min, $139 \pm 29$ min, and $164 \pm 52$ min, respectively.

In addition, the use of Ca-DPA-activated spore suspensions was tested in test systems comprising *G. stearothermophilus* C953 (DSM 1550) or *A. pallidus* MHI 3391, wherein a three-hour pre-incubation was omitted, as well as Ca-DPA-activated suspensions in which a centrifugation step was performed after pre-incubation to remove the Ca-DPA-containing supernatant. A significantly longer incubation period was observed in these preparations (data not shown).

## EXAMPLE 7 - microbial test system

### Materials & Methods

*Production of a microbial test system- embedding of spores into the growth medium*

Microbial test systems were prepared according to methods L 01.01-5 (January 2012) and L 01.00-11 (December 2002) ASU. 6.85 g BRT basis medium comprising about 1.5 g/l meat extract, about 3.0 g/l yeast extract, about 4.0 g/l casein peptone, about 6.0 g/l meat peptone, about 1.0 g/l D(+)-glucose, about 10 ml/l trimethoprim solution, and about 10 to about 15 g/l were dissolved in 250 ml distilled water and autoclaved.

The medium was tempered to 65 °C. 6.25 ml of sterile-filtered brilliant black solution (4 mg/ml, in dist. aqua) and medium additives and the spore suspension was added. The volume of spore suspension to be used was calculated on the basis of the desired spore number [cfu/ml] and the determined spore number of the spore suspension. The spore suspension was adjusted to a volume corresponding to 0.4 to 6 % v/v of the growth medium by dilution in Ringer's solution or by centrifugation ($6,000 \times$ g, RT, 10 min).

100 μl growth medium per well was aliquoted into 96-well microtiter plates, the test system plates were solidified under the sterile bench for at least 30 min, the test system plates were sealed with adhesive foil and stored at 4 °C with the bottom surface facing upwards.

*Cultivation of spore suspensions in microbial test systems*

Test systems with *Aeribacillus pallidus* MHI 3391 embedded in a growth medium has been incubated at 61 ± 1 °C until complete color change of negative control cavities from blue to yellow.

*Determination of specificity*

Cavities that were not or incompletely discolorized were considered false-positive test results.
Since no further tests were carried out that would allow inhibitor identification and quantification, the presence of inhibitor-free samples was assumed.
Specificity was calculated using the following formula:

$$Specificity\;[\%] = \frac{results_{total} - results_{false\;positive}}{results_{total}} \times 100$$

*Determination of an influence of different incubation temperatures*

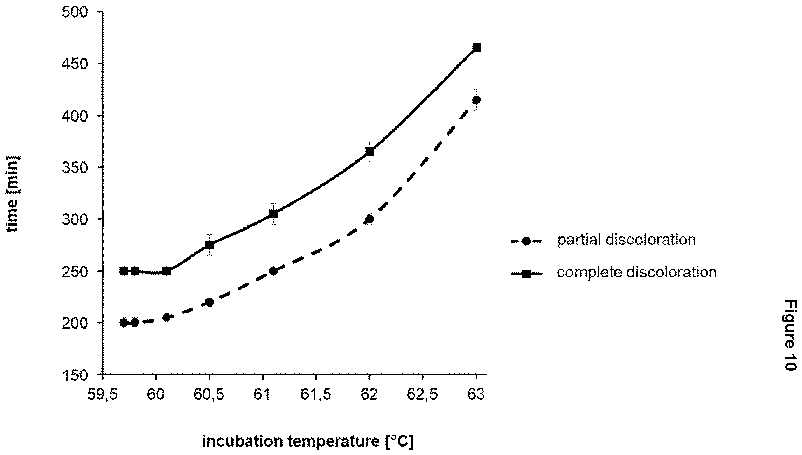

50 μl of each spore suspension of *Aeribacillus pallidus* MHI 3391 embedded in a growth medium was filled into the wells of a 96 well PCR plate. 50 μl of the negative control (UHT milk, fat content of 3.5%) was filled into each well and incubated in a thermocycler for a period of 8 h with a temperature gradient set at a constant level. The temperature gradient covered a temperature range from 59.7 °C to 65.3 °C. Visual assessment of the discoloration of individual wells was performed at intervals of 10 min. The curves are shown in Figure 10.

## Production and evaluation of a microbial test system for detecting an inhibitor in milk

*A. pallidus* MHI 3391 was used as the indicator bacteria, endospores were used with a spore count of $1.56 \times 10^7$ cfu/ml growth medium, the growth medium used was based on BRT basis medium comprising a brilliant black concentration of 100 mg/l, 100 μg/l MOX and 8000 μg/l 8-hydroxyquinoline, adjusted to a pH of 8.5. In the following, this test system will be referred to as FQ-BRT. The incubation of the FQ-BRT was carried out in a water bath at 61 ± 1 °C.

*Detection limits*

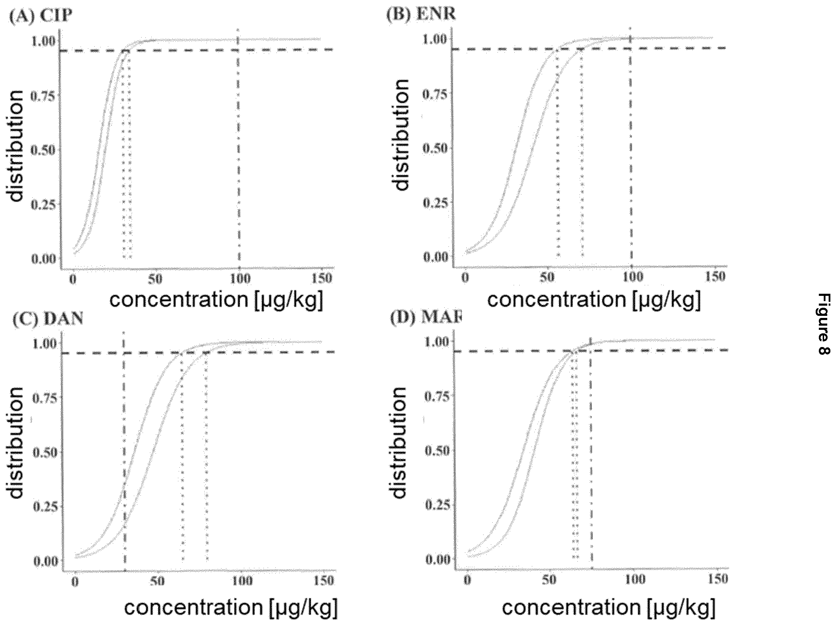

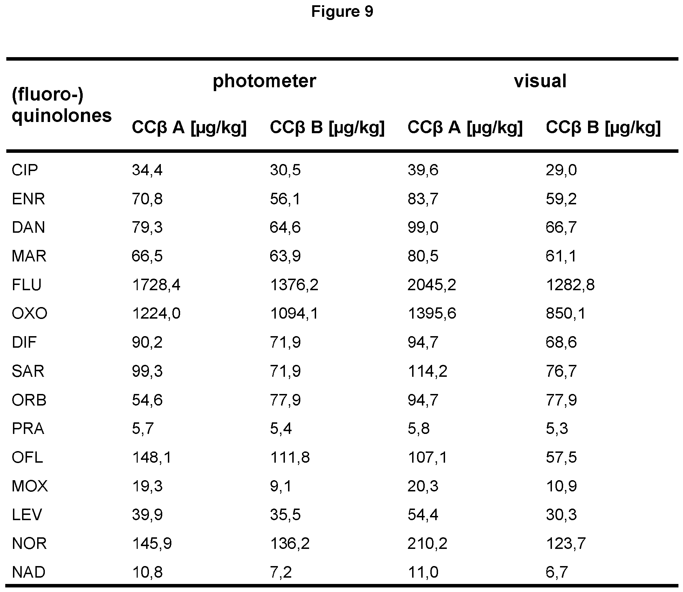

Detection limits (CCβ) of the FQ-BRT of inhibitors used for therapeutic purposes in veterinary and human medicine were calculated using a logistic regression model, based on 60 measurements of the inhibitors CIP, ENR, DAN and MAR and 18 measurements of eleven other fluoroquinolones.
The detection limits determined during the photometric and visual evaluation of test plates are shown in Figure 8 based on positive and negative control (CCβ A and CCβ B) derived from quality curves generated using the logistic regression model.
For CIP, ENR, DAN and MAR, quality curves are shown in Figure 9, which were derived from the test results after photometric evaluation. Calculated detection limits for CIP were determined in the range between 29.0 and 39.6 μg/kg, depending on the reading method and result evaluation. Detection limits for ENR were determined at concentrations ranging from 56.0 to 83.7 μg/kg. Thus, detection limits for both substances are lower as the MRL and lower as the detection limits of 100 μg/kg each set by the RohmilchGütV.

*Specificity*

The specificity of the FQ-BRT was determined using 120 randomly selected tank milk samples, examined in parallel on FQ-BRT plates from three different production batches, each in duplicate. These samples were collected in different dairy farms in southern Germany
A specificity of 98.33% for visual evaluation and 97.36% for photometric evaluation was determined for the FQ-BRT based on the tank milk samples tested. No sample showed more than one unstained or incompletely discolorized cavity in the FQ-BRT when tested in six-fold.

*Robustness of test results*

Robustness or stability of a method is understood as its ability to deliver consistent results under varying conditions.

Incubation of BRT systems was carried out in all experiments until complete discoloration of all wells of the negative control in order to determine detection sensitivities and compare incubation times.

For testing a large number of samples in routine laboratories, incubation of agar diffusion tests often occurs beyond color change.

Therefore, effects of extended incubation time of FQ-BRT on detection sensitivities of CIP, ENR and MAR were evaluated using spiked milk samples. Discoloration of the negative control on test plates of the FQ-BRT using an UHT milk negative control was observed after an incubation of 302 ± 35 min.

No changes in detection sensitivities were observed with incubation extended by 30 min beyond the time of discoloration of the negative control. Extending the incubation period by 60, 120, and 180 min resulted in the discoloration of additional wells of low dilution levels of all fluoroquinolones. Detection of CIP and ENR at concentrations of 50 $\mu$g/kg and 100 $\mu$g/kg ENR, respectively, and thus in conformance with the detection level, was also possible when plates were incubated for additional four hours, relative to the discoloration time of the negative control. The detection of MAR at the detection level of 75 $\mu$g/kg was observed when the test plates were incubated for additional two hours. When the incubation of the test plates was extended by three hours, a decrease in the detection sensitivity for MAR to 100 $\mu$g/kg was observed.

After overnight incubation of the test plates in a water bath, a positive test result was only detectable in spiked samples of 400 $\mu$g/kg. CIP, ENR and MAR can be detected at a detection level of 100 $\mu$g/kg and 75 $\mu$g/kg, respectively, even after an extension of the incubation time by two hours.

*Influence of different incubation temperatures*

With increasing incubation temperature, a continuous increase in the discoloration time of the test mixtures was observed. When the incubation temperature was increased from 60.1 °C to 61.1 °C, complete discoloration of the cavities was observed 50 ± 10 min later. Cavities incubated at temperatures of 63.8 °C and higher did not show any discoloration within the observation period of 8 h. Figure 10 shows the effect of deviating incubation temperatures on the growth and the associated brilliant black reduction of the indicator strain A. *pallidus* MHI 3391.

*Detection of inhibitors of other substance classes*

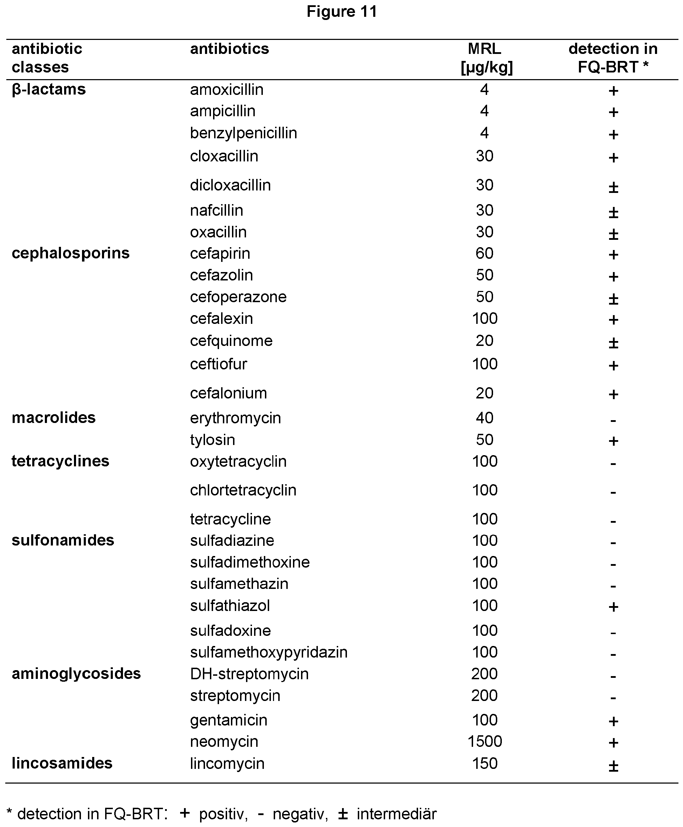

Figure 11 shows which of the 32 tested inhibitors could be detected at concentrations of their respective MRL using *A. pallidus* MHI 3391 as an indicator bacteria.

The majority of inhibitors belonging to the group of β-lactam antibiotics led to clearly positive test results in the FQ-BRT at concentrations at the level of the respective MRL. Spiked milk samples comprising the inhibitors dicloxacillin, nafcillin, oxacillin, cefoperazone and cefquinome showed intermediate test results.

Spiked milk samples comprising the inhibitor tylosin, a macrolide antibiotic, or the inhibitors gentamicin and neomycin, aminoglycoside antibiotics, caused a positive test result.

**Claims**

1. A microbial test system for detecting an inhibitor of bacterial growth in milk comprising indicator bacteria of the species *Aeribacillus pallidus* embedded in a growth medium,

   wherein the growth medium contains a dye capable of changing color upon growth of the indicator bacteria in the growth medium; and
   wherein the growth medium contains 8-hydroxyquinoline.

2. The microbial test system of claim 1, wherein the growth medium contains moxifloxacin and 8-hydroxyquinoline.

3. Use of indicator bacteria of the species *Aeribacillus pallidus* for detecting an inhibitor of bacterial growth in milk.

4. A method for detecting an inhibitor of bacterial growth in milk comprising the steps of:

   a) contacting a milk sample with indicator bacteria of the species *Aeribacillus pallidus,* wherein the indicator bacteria are embedded in a growth medium,

b) incubating the indicator bacteria in the growth medium under conditions suitable for growth of the indicator bacteria, and

c) determining the growth of the indicator bacteria in the growth medium, wherein the inhibitor is present in the milk sample if the growth of the bacteria is inhibited.

5. A test kit for detecting an inhibitor of bacterial growth in milk comprising indicator bacteria of the species *Aeribacillus pallidus* and a growth medium,

wherein the growth medium contains a dye capable of changing color upon growth of the indicator bacteria in the growth medium; and

wherein the growth medium further contains 8-hydroxyquinoline.

6. The microbial test system, the use, the method or the test kit of any one of the preceding claims wherein the indicator bacteria of the species *Aeribacillus pallidus* are from the strain *Aeribacillus pallidus* MHI 3391, deposited as DSM34046.

7. The microbial test system, the use, the method or the test kit of any one of the preceding claims, wherein the inhibitor of bacterial growth is an antibiotic.

8. The microbial test system, the use, the method or the test kit of any one of the preceding claims, wherein as little as about 100 $\mu$g ciprofloxacin per about 1 kg milk and about 75 $\mu$g enrofloxacin or marbofloxacin per about 1 kg milk can be detected.

9. A growth medium for detecting an inhibitor of bacterial growth in milk comprising

• a dye capable of changing color upon growth of an indicator bacteria in the growth medium, preferably brilliant black BN, and

• moxifloxacin and 8-hydroxyquinoline

10. The growth medium of claim 9, further comprising at least one of the following:

• meat extract,
• yeast extract,
• casein peptone, tryptic digested,
• meat peptone, tryptic digested,
• D(+)-glucose,
• trimethoprim solution, and
• agar.

11. The growth medium of claim 10, wherein the meat extract is present at about 1.5 g/l, the yeast extract at about 3.0 g/l, the casein peptone at about 4.0 g/l, the meat peptone at about 6.0 g/l, the D(+)-glucose at about 1.0 g/l, the trimethoprim solution at about 10 ml/l, and/or the agar at about 10 to about 15 g/l.

12. The growth medium of any one of the preceding claims, wherein the growth medium has a pH value of about pH 5.5 - pH 9.5.

13. The growth medium of any one of the preceding claims, wherein moxifloxacin is present at about 100 $\mu$g/l - 150 $\mu$g/l.

14. The growth medium of any one of the preceding claims, wherein 8-hydroxyquinoline is present at about 2000 $\mu$g/l - 8000 $\mu$g/l.

**Patentansprüche**

1. Mikrobielles Testsystem zum Nachweis eines Hemmstoffs für bakterielles Wachstum in Milch, umfassend

Indikatorbakterien der Spezies *Aeribacillus pallidus,* eingebettet in ein Wachstumsmedium,

wobei das Wachstumsmedium einen Farbstoff enthält, der in der Lage ist, seine Farbe bei Wachstum der Indikatorbakterien in dem Wachstumsmedium zu ändern; und
wobei das Wachstumsmedium 8-Hydroxychinolin enthält.

2. Das mikrobielle Testsystem nach Anspruch 1, wobei das Wachstumsmedium Moxifloxacin und 8-Hydroxychinolin enthält.

3. Verwendung von Indikatorbakterien der Spezies *Aeribacillus pallidus* zum Nachweis eines Inhibitors des bakteriellen Wachstums in Milch.

4. Verfahren zum Nachweis eines Inhibitors des bakteriellen Wachstums in Milch, umfassend die Schritte:

   a) Inkontaktbringen einer Milchprobe mit Indikatorbakterien der Spezies *Aeribacillus pallidus,* wobei die Indikatorbakterien in ein Wachstumsmedium eingebettet sind,
   b) Inkubieren der Indikatorbakterien in dem Wachstumsmedium unter Bedingungen, die für das Wachstum der Indikatorbakterien geeignet sind, und
   c) Bestimmen des Wachstums der Indikatorbakterien in dem Wachstumsmedium, wobei der Inhibitor in der Milchprobe vorhanden ist, wenn das Wachstum der Bakterien gehemmt ist.

5. Testkit zum Nachweis eines Inhibitors bakteriellen Wachstums in Milch, umfassend Indikatorbakterien der Spezies *Aeribacillus pallidus* und ein Wachstumsmedium,

   wobei das Wachstumsmedium einen Farbstoff enthält, der in der Lage ist, seine Farbe bei Wachstum der Indikatorbakterien in dem Wachstumsmedium zu ändern; und
   wobei das Wachstumsmedium zusätzlich 8-Hydroxychinolin enthält.

6. Das mikrobielle Testsystem, die Verwendung, das Verfahren oder der Testkit nach einem der vorhergehenden Ansprüche, wobei die Indikatorbakterien der Spezies *Aeribacillus pallidus* von dem Stamm *Aeribacillus pallidus* MHI 3391 sind.

7. Das mikrobielle Testsystem, die Verwendung, das Verfahren oder der Testkit nach einem der vorstehenden Ansprüche, wobei der Inhibitor des bakteriellen Wachstums ein Antibiotikum ist.

8. Das mikrobielle Testsystem, die Verwendung, das Verfahren oder der Testkit nach einem der vorhergehenden Ansprüche, bei dem schon etwa 100 $\mu$g Ciprofloxacin pro etwa 1 kg Milch und etwa 75 $\mu$g Enrofloxacin oder Marbofloxacin pro etwa 1 kg Milch nachgewiesen werden können.

9. Wachstumsmedium zum Nachweis eines Inhibitors bakteriellen Wachstums in Milch, umfassend

   • einen Farbstoff, der in der Lage ist, beim Wachstum eines Indikatorbakteriums in dem Wachstumsmedium die Farbe zu ändern, vorzugsweise Brillantschwarz BN, und
   • Moxifloxacin und 8-Hydroxychinolin.

10. Das Wachstumsmedium nach Anspruch 9, das ferner mindestens eines der folgenden enthält:

    • Fleischextrakt,
    • Hefeextrakt,
    • Kaseinpepton, tryptisch verdaut,
    • Fleischpepton, tryptisch verdaut,
    • D(+)-Glucose,
    • Trimethoprim-Lösung und
    • Agar.

11. Das Wachstumsmedium nach Anspruch 10, wobei der Fleischextrakt in einer Menge von etwa 1,5 g/l, der Hefeextrakt in einer Menge von etwa 3,0 g/l, das Kaseinpepton in einer Menge von etwa 4,0 g/l, das Fleischpepton in einer Menge von etwa 6,0 g/l, die D(+)-Glucose in einer Menge von etwa 1,0 g/l, die Trimethoprimlösung in einer Menge von etwa 10 ml/l und/oder der Agar in einer Menge von etwa 10 bis etwa 15 g/l vorhanden ist.

12. Das Wachstumsmedium nach einem der vorhergehenden Ansprüche, wobei das Wachstumsmedium einen pH-Wert von etwa pH 5,5 bis pH 9,5 aufweist.

13. Das Wachstumsmedium nach einem der vorangehenden Ansprüche, wobei Moxifloxacin in einer Menge von etwa 100 $\mu$g/l bis 150 $\mu$g/l vorhanden ist.

14. Das Wachstumsmedium nach einem der vorangehenden Ansprüche, wobei 8-Hydroxychinolin in einer Menge von etwa 2000 $\mu$g/l - 8000 $\mu$g/l vorhanden ist.

**Revendications**

1. Système d'essai microbien pour la détection d'un inhibiteur de croissance bactérienne dans le lait comprenant des bactéries indicatrices de l'espèce *Aeribacillus pallidus* incorporées dans un milieu de croissance, dans lequel le milieu de croissance contient un colorant capable de changer de couleur lors de la croissance des bactéries indicatrices dans le milieu de croissance ; et
dans lequel le milieu de croissance contient de la 8-hydroxyquinoléine.

2. Système d'essai microbien selon la revendication 1, dans lequel le milieu de croissance contient de la moxifloxacine et de la 8-hydroxyquinoléine.

3. Utilisation de bactéries indicatrices de l'espèce *Aeribacillus pallidus* pour la détection d'un inhibiteur de croissance bactérienne dans le lait.

4. Méthode de détection d'un inhibiteur de croissance bactérienne dans le lait comprenant les étapes de :

a) mise en contact d'un échantillon de lait avec des bactéries indicatrices de l'espèce *Aeribacillus pallidus,* dans lequel les bactéries indicatrices sont incorporées dans un milieu de croissance,
b) incubation des bactéries indicatrices dans le milieu de croissance dans des conditions appropriées pour la croissance des bactéries indicatrices, et
c) détermination de la croissance des bactéries indicatrices dans le milieu de croissance, dans lequel l'inhibiteur est présent dans l'échantillon de lait si la croissance des bactéries est inhibée.

5. Trousse d'essai pour la détection d'un inhibiteur de croissance bactérienne dans le lait comprenant des bactéries indicatrices de l'espèce *Aeribacillus pallidus* et un milieu de croissance,

dans lequel le milieu de croissance contient un colorant capable de changer de couleur lors de la croissance des bactéries indicatrices dans le milieu de croissance ; et
dans lequel le milieu de croissance contient en outre de la 8-hydroxyquinoléine.

6. Système d'essai microbien, utilisation, méthode ou trousse d'essai selon l'une quelconque des revendications précédentes dans lesquels les bactéries indicatrices de l'espèce *Aeribacillus pallidus* proviennent de la souche *Aeribacillus pallidus* MHI 3391, déposée sous DSM34046.

7. Système d'essai microbien, utilisation, méthode ou trousse d'essai selon l'une quelconque des revendications précédentes, dans lesquels l'inhibiteur de croissance bactérienne est un antibiotique.

8. Système d'essai microbien, utilisation, méthode ou trousse d'essai selon l'une quelconque des revendications précédentes, dans lesquels on peut détecter aussi peu que 100 $\mu$g environ de ciprofloxacine pour environ 1 kg de lait et 75 $\mu$g environ d'enrofloxacine ou de marbofloxacine pour environ 1 kg de lait.

9. Milieu de croissance pour la détection d'un inhibiteur de croissance bactérienne dans le lait comprenant

• un colorant capable de changer de couleur lors de la croissance d'une bactérie indicatrice dans le milieu de croissance, de préférence le noir brillant BN, et
• de la moxifloxacine et de la 8-hydroxyquinoléine.

10. Milieu de croissance selon la revendication 9, comprenant en outre au moins un des suivants :

- extrait de viande,
- extrait de levure,
- peptone de caséine, obtenue par digestion trypsique,
- peptone de viande, obtenue par digestion trypsique,
- D(+)-glucose,
- solution de triméthoprime, et
- agar-agar.

11. Milieu de croissance selon la revendication 10, dans lequel l'extrait de viande est présent à environ 1,5 g/l, l'extrait de levure à environ 3,0 g/l, la peptone de caséine à environ 4,0 g/l, la peptone de viande à environ 6,0 g/l, le D(+)-glucose à environ 1,0 g/l, la solution de triméthoprime à environ 10 ml/l, et/ou l'agar-agar à environ 10 à environ 15 g/l.

12. Milieu de croissance selon l'une quelconque des revendications précédentes, dans lequel le milieu de croissance a une valeur de pH d'environ pH 5,5 - pH 9,5.

13. Milieu de croissance selon l'une quelconque des revendications précédentes, dans lequel la moxifloxacine est présente à environ 100 $\mu$g/l - 150 $\mu$g/l.

14. Milieu de croissance selon l'une quelconque des revendications précédentes, dans lequel la 8-hydroxyquinoléine est présente à environ 2000 $\mu$g/l - 8000 $\mu$g/l.

Figure 1

Figure 2

| strain | sporulation medium | incubation time [h] | sporulation rate [%] | avarage spore count [cfu/ml] |
|---|---|---|---|---|
| *G. stearothermophilus* C953 (DSM 1550) | milk agar | 48 | > 95 | $1,72 \times 10^8$ |
| *G. stearothermophilus* MHI 3387 | TSA | 24 | 80 | $5,41 \times 10^8$ |
| *A. pallidus* MHI 3390 | TSA | 72 | 80 | $8,34 \times 10^8$ |
| *A. pallidus* MHI 3391 | TSA | 24 | 80 | $1,26 \times 10^{10}$ |
| *A. thermoaerophilus* MHI 3397 | milk agar | 72 | > 95 | $3,06 \times 10^8$ |
| *A. flavithermus* MHI 3392 | TSA | 48 | 90 | $3,65 \times 10^6$ |
| *B. thermoamylovorans* MHI 3373 | Schäffer agar | 24 | 90 | $1,47 \times 10^{10}$ |

Figure 3

**Figure 4**

| antibiotic classes | antibiotics | methods | MIC [µg/l] | | |
| --- | --- | --- | --- | --- | --- |
| | | | *G. stearo-thermophilus* C953 (DSM 1550) | *G. stearothermophilus* MHI 3387 | *A. pallidus* MHI 3391 |
| **fluoroquinolones** | CIP | MD | 125 | 62,5 | 31,25 |
| | | GD | 125 | 64 | 32 |
| | ENR | MD | 250 | 125 | 62,5 |
| | | GD | 190 | 190 | 32 |
| | DAN | MD | 250 | 125 | 62,5 |
| | MAR | MD | 300 | 300 | 150 |
| | | GD | 250 | 125 | 94 |
| | NOR | MD | 500 | 500 | 250 |
| | | GD | 500 | 250 | 190 |
| | LEV | MD | 250 | 125 | 62,5 |
| | MOX | MD | 125 | 62,5 | 15,6 |
| **β-lactams** | penicillin G | MD | 0,016 | 0,5 | 0,25 |
| | | GD | < 2 | 3 | 8 |
| | cefquinome | MD | 40 | 40 | 320 |
| **tetracyclines** | tetracycline | MD | 50 | 200 | 100 |
| | | GD | 64 | 32 | 94 |
| **sulfonamides** | sulfadoxine | MD | > 3200 | > 3200 | > 3200 |
| **macrolides** | erythromycin | MD | 100 | 6,25 | 800 |
| **aminoglycosides** | gentamicin | MD | 200 | > 1600 | 100 |
| **lincosamides** | lincomycin | MD | 37,5 | 75 | 600 |

Figure 5

G. stearothermophilus C953 (DSM 1550)

A. pallidus MHI 3391

Figure 6

(A) *G. stearothermophilus* C 953 (DSM 1550)

**Figure 6 (continued)**

**(B)** *A. pallidus* MHI 3391

Figure 7

**Figure 8**

Figure 9

| (fluoro-) quinolones | photometer | | visual | |
|---|---|---|---|---|
| | CCβ A [μg/kg] | CCβ B [μg/kg] | CCβ A [μg/kg] | CCβ B [μg/kg] |
| CIP | 34,4 | 30,5 | 39,6 | 29,0 |
| ENR | 70,8 | 56,1 | 83,7 | 59,2 |
| DAN | 79,3 | 64,6 | 99,0 | 66,7 |
| MAR | 66,5 | 63,9 | 80,5 | 61,1 |
| FLU | 1728,4 | 1376,2 | 2045,2 | 1282,8 |
| OXO | 1224,0 | 1094,1 | 1395,6 | 850,1 |
| DIF | 90,2 | 71,9 | 94,7 | 68,6 |
| SAR | 99,3 | 71,9 | 114,2 | 76,7 |
| ORB | 54,6 | 77,9 | 94,7 | 77,9 |
| PRA | 5,7 | 5,4 | 5,8 | 5,3 |
| OFL | 148,1 | 111,8 | 107,1 | 57,5 |
| MOX | 19,3 | 9,1 | 20,3 | 10,9 |
| LEV | 39,9 | 35,5 | 54,4 | 30,3 |
| NOR | 145,9 | 136,2 | 210,2 | 123,7 |
| NAD | 10,8 | 7,2 | 11,0 | 6,7 |

## Figure 10

Figure 11

| antibiotic classes | antibiotics | MRL [µg/kg] | detection in FQ-BRT * |
|---|---|---|---|
| β-lactams | amoxicillin | 4 | + |
| | ampicillin | 4 | + |
| | benzylpenicillin | 4 | + |
| | cloxacillin | 30 | + |
| | dicloxacillin | 30 | ± |
| | nafcillin | 30 | ± |
| | oxacillin | 30 | ± |
| cephalosporins | cefapirin | 60 | + |
| | cefazolin | 50 | + |
| | cefoperazone | 50 | ± |
| | cefalexin | 100 | + |
| | cefquinome | 20 | ± |
| | ceftiofur | 100 | + |
| | cefalonium | 20 | + |
| macrolides | erythromycin | 40 | - |
| | tylosin | 50 | + |
| tetracyclines | oxytetracyclin | 100 | - |
| | chlortetracyclin | 100 | - |
| | tetracycline | 100 | - |
| sulfonamides | sulfadiazine | 100 | - |
| | sulfadimethoxine | 100 | - |
| | sulfamethazin | 100 | - |
| | sulfathiazol | 100 | + |
| | sulfadoxine | 100 | - |
| | sulfamethoxypyridazin | 100 | - |
| aminoglycosides | DH-streptomycin | 200 | - |
| | streptomycin | 200 | - |
| | gentamicin | 100 | + |
| | neomycin | 1500 | + |
| lincosamides | lincomycin | 150 | ± |

* detection in FQ-BRT:  +  positiv,  -  negativ,  ±  intermediär

## REFERENCES CITED IN THE DESCRIPTION

**Patent documents cited in the description**

- EP 21200810 A

- FR 2882370

**Non-patent literature cited in the description**

- **MUHAMMAD ; AHMED**. Production and characterization of a new antibacterial peptide obtained from Aeribacillus pallidus SAT4. *Biotechnology Repoorts*, 2015, vol. 8, 72-80
- **HARIRCHI et al.** Efficacy of polyectremophilic Aeribacillus pallidus on bioprocessing of beet vinasse derived from ethanol industries. *Bioresource Technology*, 2020, vol. 313
- **GUNA et al.** In vitro activity of linezolid, clarithromycin and moxifloxacin against clinical isolates of Mycobacterium kansasii. *Journal of Antimicrobial Chemotherapy*, 2005, vol. 55, 950-953

- **SALA et al.** Simple Model for Testing Drugs agains Nonreplicating Mycobacterium tuberculosis. *Antimicrobial Agants and Chemotherapy*, 2010, vol. 54 (10), 4150-4158
- **WU et al.** A Novel Microbiological Method in Microtiter Plates for Screening Seven Kinds of Widely Used Antibiotics Residues in Milk, Chicken Egg and Honey. *Front. Microbiol.*, 2019, vol. 10 (436), 1-11